# EUROPEAN PATENT SPECIFICATION

(11) **EP 3 902 520 B1**
(45) Date of publication and mention of the grant of the patent: **05.02.2025**
(21) Application number: 19829655.0
(22) Date of filing: 27.12.2019
(51) Int. Cl.: A61K 9/00, A61K 33/24, A61P 35/00, A61K 33/243, A61K 9/14

(54) **KIT FOR INHALED CHEMOTHERAPY, AND TREATMENT OF LUNG CANCER WITH SAID KIT**
KIT FÜR INHALATIONSCHEMOTHERAPIE UND BEHANDLUNG VON LUNGENKREBS MIT DEM KIT
KIT POUR UNE CHIMIOTHÉRAPIE PAR INHALATION DESTINÉ AU TRAITEMENT DE CANCER DU POUMON

(30) Priority: 28.12.2018 WO PCT/EP2018/097140
(43) Date of publication of application: 03.11.2021
(73) Proprietor: Université Libre de Bruxelles, 1050 Bruxelles (BE)
(72) Inventor: AMIGHI, Karim, 1150 Brussels (BE); WAUTHOZ, Nathalie, 1180 Bruxelles (BE); ROSIÈRE, Rémi, 1170 Brussels (BE)
(74) Representative: Calysta NV
(86) International application number: PCT/EP2019/087118
(87) International publication number: WO 2020/136272

(56) References cited:
- EP-A2- 0 211 595
- US-A- 4 627 432
- US-A- 4 778 054
- US-A1- 2005 107 287
- US-A1- 2007 065 522
- US-A1- 2007 122 350
- LEVET VINCENT ET AL: "Platinum pharmacokinetics in mice following inhalation of cisplatin dry powders with different release and lung retention properties", INTERNATIONAL JOURNAL OF PHARMACEUTICS, ELSEVIER, NL, vol. 517, no. 1, 19 December 2016 (2016-12-19), pages 359 - 372, XP029878094, ISSN: 0378-5173, DOI: 10.1016/J.IJPHARM.2016.12.037
- VINCENT LEVET ET AL: "Development of controlled-release cisplatin dry powders for inhalation against lung cancers", INTERNATIONAL JOURNAL OF PHARMACEUTICS, vol. 515, no. 1-2, 11 October 2016 (2016-10-11), NL, pages 209 - 220, XP055584532, ISSN: 0378-5173, DOI: 10.1016/j.ijpharm.2016.10.019

## Description

The present invention relates to a kit for an inhaled chemotherapy, the suitable use of the kit for an inhaled chemotherapy in a polytherapy against lung cancer.

Lung cancer is the cancer with the highest prevalence and mortality in the world. In most cases, lung cancer is diagnosed at advanced stages. Therefore, the patients often present already metastases in the lungs or in other organs, i.e. extrapulmonary metastases. Treatment modalities are mostly used in combination and consist in surgery, radiotherapy, chemotherapy, targeted therapy and immunotherapy.

Chemotherapy is used in up to 60% of lung cancer patients, mainly in advanced stages of the disease. Chemotherapy is currently administered through intravenous injection or per os, i.e. systemic routes of administration, i.e. systemic chemotherapy. Chemotherapy is responsible for severe systemic toxicities due to (i) the broad distribution of the chemotherapeutic in the organism and (ii) the lack of selectivity for cancer cells. Oncologists are consequently in great need for new more efficient and better-tolerated treatment approaches.

Chemotherapy is indeed a matter of finding the highest amount of anti-neoplastic agent to be administered by reducing at a maximum level the non-reversible toxicity side effects and disagreeable side effects generated by the chemotherapy.

Accordingly, very complex therapeutic scheme are foreseen combining very often different therapeutic modalities and acts, such as for example, surgery, with a first cycle of radiotherapy, followed by complex cycles of anti-neoplastic agent injections or intravenous infusion, which can be the same ordifferent for each cycle and adapted depending on response to said therapeutic scheme and the side effect identified on the body of the patient.

Each anti-neoplastic agent or molecule has a dose limiting toxicity (DLT) such as nephrotoxicity, neurotoxicity, ... which require to introduce in the therapeutic scheme rest period allowing the patient body to recover from the adverse side effects, when not non-reversible.

Further, anti-neoplastic agent has a half-life time period which directly determine the duration of action of a drug. The half-life time period is the period of time required for the concentration or amount of drug in the body to be reduced by one-half. The half-time period of anti-neoplastic agent is typically comprised between 12 hours and 36 hours, which can be short as representing the exposition time of the human body to the beneficial effect of the anti-neoplastic agent.

However, due to the DLT of anti-neoplastic agent, the amount of administered doses during several administering periods (i.e., a cycle) remains limited and should be separated one to each other, as said previously, by off-period (see for example US2007/0065522-§0010).

The combination of the half-life period together with the dose limiting toxicities, together with the fact that when the anti-neoplastic agent is administered by injection or intravenous infusion, the concentration of the anti-neoplastic agent which reach efficiently the solid tumor site is low, with limited effect on the tumor itself and high systemic toxicity for the patient body.

Further another constrain of anti-neoplastic agent is the cumulative dose which is the total dose resulting from repeated exposures to the anti-neoplastic agent of the same part of the body or of the whole body.

For cisplatin, administered through intravenous route, the cumulative dose is 300 mg/m² (m² of the human body) within 4 to 6 cycles.

Those considerations yielded researchers to develop more targeted therapies and on-site injection/infusions.

One approach is to deliver chemotherapy by means of the pulmonary route (see for example US2007/0065522), i.e. by inhalation. Inhalation allows the administration of high drug doses directly to lung tumors without prior distribution in the organism. The severe systemic toxicities are consequently reduced.

However, local adverse effects linked to the pulmonary route can also occur depending on the drug candidate, its formulation and the related inhalation device. Moreover, the higher financial costs (due to particular hospital facilities and inhalation procedure to limit environmental contamination), in addition to the lack of drastic improvement in the benefit/risk ratio in both preclinical and clinical evaluations, make that pulmonary route has been underexploited in lung tumor therapy.

While chemotherapy delivered through the pulmonary route may offer strong perspectives, there is a need to provide candidate which can improve the benefit/risk ratio in both preclinical and clinical evaluation and are susceptible to provide a candidate where the benefits of chemotherapy will outweigh the adverse effect together with improving the life quality.

Inhaled therapy is of different kind and one can distinguish nebulizers from inhalers as distinct manner to deliver active substances to the respiratory tract. Inhalers can also be of different type and dry powder inhalers (DPI) are one of them.

Compared to nebulizers, dry powder inhalers (DPI) are well-adapted to chemotherapy. They allow high doses of active substances, but also poorly water-soluble compounds (i.e. most chemotherapeutics in cancer) to be administered directly into the lungs. Moreover, DPI limit environmental contamination by the aerosol due to (i) their activation and drive by the patient's inspiratory flow only and (ii) negligible exhaled drug doses. Finally, DPI can be designed as single-use disposable devices.

However, conventional immediate-release DPI formulations might nevertheless lead to too short residence in the lungs and to poor local tolerance. These may result from the fast dissolution of drug particles and high peak concentrations of the anticancer drug in lung fluids after lung deposition.

There is therefore a need to develop pulmonary route for chemotherapy and to adapt deeply current inhalation devices and formulations used in clinical trials to deliver effective anticancer therapies to tumor-bearing lungs.

In that context, controlled-release cisplatin-based DPI formulations were developed with high drug loading and high fine particle fraction (Levet et al, Int J Pharm 2016). The formulations presented controlled-release and prolonged lung retention abilities leading to low systemic distribution (Levet et al, Int J Pharm 2017). According to this document, in a lung cancer mice model, this approach led to comparable tumor response in vivo than the intravenous regimen at half dose [dose (1.0 mg/kg vs 0.5 mg/kg, respectively)]. The intravenous regimen gives good results for extra pulmonary metastases while inhaled cisplatin seemed more active against primary lung tumors.

DPI formulations composed of paclitaxel-based nanocarriers were also developed with increased residence time in the lungs, limited systemic distribution and aimed specifically at lung cancer cells by targeting the folate receptor (FR) (Rosière et al, Int J Pharm 2016; Rosière et al, Mol Pharm, 2018). FR, especially FR-a, is overexpressed on the cancer cell surfaces in many lung tumors (i.e. more than 70% of adenocarcinomas) and is a promising membrane receptor to target in lung cancer. Significantly longer survival rates were observed for the FR-targeted inhaled treatments in combination with intravenous Taxol^{®} (the commercial form of paclitaxel), than for intravenous Taxol^{®} alone in a lung cancer mice model.

To resume, although the use of DPI chemotherapy led to encouraging preclinical results in terms of pharmacokinetic profiles and safety, efficacy is still rather limited.

The present invention encounters to solve at least a part of these drawbacks by providing a kit, for example to be used in a polytherapy, for an inhaled chemotherapy.

The kit according to the present invention is characterized in that it comprises:
a) a series of single hermetically packaged therapeutic doses of a dry powder for inhalation containing at least one anti-neoplastic agent chosen in the group consisting of Platinum drugs such as cisplatin, carboplatin, oxaliplatin and nedaplatin with a lipid matrix and optionally one or more excipient, such as a PEGylated excipient and/or a carrier
b) a series of disposable dry powder inhaler devices,
   wherein
   i) each single hermetically packaged therapeutic dose having a closed state and an open state,
   ii) each disposable dry powder inhaler device having a closed position and an open position,
   iii) each single hermetically packaged therapeutic dose being provided for being accommodated in said closed state in one disposable dry powder inhaler device of said series of disposable dry powder inhaler devices, and
   iv) each single disposable dry powder inhaler device is provided to be in said closed position before or once each single hermetically packaged therapeutic dose become in said open state, wherein each disposable dry powder inhaler device comprises a dispersion chamber in fluid connection with an inhalation channel and connected to a loading chamber for accommodating said single hermetically packaged therapeutic dose inside said disposable dry powder inhaler device, said loading chamber comprising activation means having an activated position and a rest position, said activated position being provided to open said single hermetically packaged therapeutic dose and locking means provided to prevent extraction of the single hermetically packaged therapeutic dose once said activation means have been moved to said activated position, or said activated position being provided to open said single hermetically packaged therapeutic dose and locking means having an open position and a lock position provided to prevent extraction of the single hermetically packaged therapeutic dose, said activation means being provided to be able to be moved to said activated position only when said locking means are in said lock position, or said disposable dry powder inhaler device comprising closing means provided to isolate the outside environment from the inside of the disposable dry powder inhaler device, in particular by closing the inhalation channel.

By the terms " one or more excipient", it is meant a compound selected from sugar alcohols; polyols (such as sorbitol, mannitol and xylitol); crystalline sugars including monosaccharides (such as glucose, arabinose) and disaccharides (such as lactose, maltose, saccharose, dextrose, trehalose, maltitol); inorganic salts (such as sodium chloride and calcium carbonate); organic salts (such as sodium lactate, potassium or sodium phosphate, sodium citrate, urea); polysaccharides (such as dextran, chitosan, starch, cellulose, hyaluronic acid, and their derivatives); oligosaccharides (such as cyclodextrins and dextrins); titanium dioxide; silicone dioxide; magnesium stearate; lecithin; amino acids (such as leucine, isoleucine, histidine, threonine, lysine, valine, methionine, phenylalanine); derivatives of an amino acid (such as acesulfame K, aspartame); lauric acid or derivatives (such as esters and salts); palmitic acid or derivatives (such as esters and salts); stearic acid or derivatives (such as esters and salts); erucic acid or derivatives (such as esters and salts); behenic acid or derivatives (such as esters and salts); sodium stearyl fumarate; sodium stearyl lactylate; phosphatidylcholines; phosphatidylglycerols; natural and synthetic lung surfactants; lauric acid and its salts (such as sodium lauryl sulphate, magnesium lauryl sulphate); triglycerides; sugar esters; phospholipids; cholesterol; talc.

In certain preferred embodiments, the excipient is mannitol, dextran, or lactose.

In certain preferred embodiments, the excipient is phospholipids or cholesterol.

In certain preferred embodiments, the excipient is mannitol, dextran, hyaluronic acid, lactose, phospholipids, or cholesterol.

In certain preferred embodiments, the excipient is mannitol, dextran, phospholipids, or cholesterol.

In certain embodiments, said at least one excipient is a carrier.

By the terms "single hermetically packaged doses", it is meant within the meaning of the present invention that the single hermetically packaged doses of the neoplastic agent under the form of a dry powder for inhalation is filled in a capsule (e.g. HPMC or gelatin capsule) sealed in a blister (e.g. aluminium-aluminium blister) or is directly filled in a blister, which is the sealed dose, i.e. the single hermetically packaged dose. When the single hermetically packaged doses of the neoplastic agent under the form of a dry powder for inhalation is filled in a capsule, the closed state of the single hermetically packaged doses means the capsule is closed and the blister is closed or the capsule is closed and the blister is open. The single hermetically packaged dose is in open state when the capsule is open for inhalation purposes, i.e. for example when an orifice has been pierced.

Within the meaning of the present invention, the said one disposable dry powder inhaler device is a capsule-based or a blister-based device that is sealed after use, i.e. after the single hermetically packaged dose has been in the open state, such as after the administration of a single dose by the patient, preferably assisted by a nurse or the medical staff.

The device can be sealed by avoiding re-opening, capping the drug exit, blocking any air exit through one-way valves, or simply introducing it into a hermetic bag after use.

The said one disposable dry powder inhaler device has a specific use procedure to limit the contamination during its use and is hermetically packaged after the use (for instance contained in a hermetic bag) in order to be eliminated as a waste using an appropriate procedure.

Preferably, the disposable dry powder device of the present invention is a disposable single dose blister-based device which is hermetically packaged (for instance, contained in a hermetic bag) in close position and already containing the single drug dose filled in in a blister in said close state. The single hermetically packaged dose reaching its open state just before its use (for instance by piercing or opening the blister), so the patient can inhale the dose contained inside the blister before the device being eliminated as explained before. The hermetic bag in which the device can be packaged before and/or after use can be the same or different.

In another preferred variant according to the present invention, the single hermetically packaged dose under the form of the blister containing the single dose and the disposable dry power blister-based device are both tightly (sealedly) packaged separately and the blister is inserted in the device just before the administration of the dose.

In a further preferred variant according to the present invention, preferably, the disposable dry powder device of the present invention is a disposable single dose capsule-based device which is hermetically packaged (i.e. contained in a hermetic bag) in close position and already containing the single drug dose filled in a capsule in said close state. The single hermetically packaged dose reaching its open state just before its use (e.g. by piercing or opening the capsule), so the patient can inhale the dose contained inside the capsule before the device being eliminated as explained before. The hermetic bag in which the device can be packaged before and/or after use can be the same or different.

In yet another preferred variant according to the present invention, the single hermetically packaged dose under the form of the capsule containing the single dose and the disposable and tight dry power capsule-based device are both tightly (sealedly) packaged separately and the capsule is inserted in the device just before the administration of the dose.

According to the present invention, the capsule-based device can be in a partially open/closed position when the single hermetically package dose is in the closed state. However, once the capsule-based device is closed by the patient/nurse after the insertion of the capsule, the device is definitely closed (i.e. a re-opening of the device is not possible) and the capsule is open for having the single hermetically packaged dose in open state), so the patient can inhale the dose contained inside the capsule before being eliminated together with the capsule-based device as explained before.

This is again to allow to limit the contamination by the anticancer drug to patient, nurse and environment during the use (i.e. re-open the device to remove the pierced/opened capsule, re-use several times the same device containing a fine powder, etc.).

In still another alternative according to the present invention, the single hermetically packaged dose, i.e. the capsule containing the single therapeutic dose in said closed state and the capsule-based disposable dry powder inhaler device are tightly (sealedly) packaged separately. The capsule is inserted in the device just before the administration of the dose. The device can be opened by the patient/nurse to allow the capsules filling. Once the capsule is adequately placed in the device capsule loading chamber in the close state, the capsule-based device is closed, and the opening of the capsule provokes the definitive closure of the device. Again, the device should be disposable and be packaged after a single-use, preferably after setting the cap on the device (cap sealing position) in a specific tight bag in order to be eliminated as a waste.

Whatever the type of the disposable device and the single hermetically packaged dose used according to the present invention, when the device is activated (e.g. by piercing/opening the capsule or the blister), the inhalation procedure by the patient can be performed one time or repeated sequentially one or two times to ensure that the whole dose contained in the device is inhaled.

The present invention, through providing a kit for inhaled chemotherapy comprising a series of single hermetically packaged therapeutic doses of at least one anti-neoplastic agent and a series of disposable dry powder inhaler devices allow to dispose of a fully secured chemotherapy in terms of environmental contamination of cytotoxic agent in the surrounding atmosphere, thereby drastically increasing the comfort of the patient and offering many possibilities for an ambulatory treatment and/or a home therapy.

It has been indeed foreseen according to the present invention a kit comprising a series of single hermetically packaged therapeutic doses of at least one anti-neoplastic agent together with a series of disposable dry powder inhaler devices, where each single hermetically packaged therapeutic doses of at least one anti-neoplastic agent will be used in a new and clean disposable dry powder inhaler device, thereby preventing the escape of cytotoxic substance in the surrounding environment as a new disposable dry powder inhaler device will be used for each single hermetically packaged therapeutic doses of at least one anti-neoplastic agent. This means that after its use, the disposable dry powder inhaler devices will be properly and immediately discarded to be then isolated in a container for cytotoxic waste to be incinerated and that for the next use, when manipulation has to be foreseen to introduce the new single hermetically packaged therapeutic doses of at least one anti-neoplastic agent in the disposable dry powder inhaler devices, the disposable dry powder inhaler devices has never been in contact with cytotoxic agent and the manipulation will never cause any contamination of the surrounding atmosphere. This allows the patient to take their therapy at home since the risk of having cytotoxic anti-neoplastic agent in the atmosphere during the manipulation of the dry powder inhaler is drastically reduced.

It is contemplated according to the present invention that this will help drastically patient suffering from lung cancer as it has been recognized that long term and/or repetitive stay(s) in hospital environment is impacting negatively the ability to recover of the patient.

To ensure no dispersion of cytotoxic substance in the surrounding atmosphere occurs, the disposable dry powder inhaler devices has an open position and a closed position and each single hermetically packaged therapeutic dose has a close state and an open state.

In the kit for inhaled chemotherapy according to the present invention, each single hermetically packaged therapeutic dose is provided for being accommodated in said closed state in one disposable dry powder inhaler device of said series of disposable dry powder inhaler devices and each single hermetically packaged therapeutic dose being also provided for becoming in said open state only when said one disposable said tight dry powder inhaler device is in said closed position.

This allows to make sure the single hermetically packaged therapeutic doses of at least one anti-neoplastic agent is not to be open if not fully accommodated in the disposable dry powder inhaler device and isolated from the surrounding environment.

In the kit according to the present invention, said single hermetically packaged therapeutic dose is a capsule or a blister.

In a preferred embodiment of the kit according to the present invention, the lipid matrix is a pure or a mixture of triglycerides, such as for example tristearin, hydrogenated vegetal oil (e.g. hydrogenated castor oil) or any hydrophobic glyceride derivative having values of Log P higher than 5, preferably comprised between 14 and 25, more particularly between 18 and 25 and preferably around 20, as measured using the shake-flask method.

According to the present invention, said lipid matrix comprises a pure or a mixture of triglycerides having a melting point temperature higher than or equal to 40°C, preferably higher than or equal to 60°C, more preferably higher than or equal to 75°C.

In another preferred embodiment of the present invention, the PEGylated excipient is derived from vitamin E or from phospholipids, such as tocopheryl polyethylene glycol succinate (TPGS) or distearoyl phosphoethanolamine polyethylene glycol 2000 (DSPE-mPEG-2000).

In the kit for a inhaled chemotherapy according to the present invention, each disposable dry powder inhaler device comprises a dispersion chamber in fluid connection with an inhalation channel and connected to a loading chamber for accommodating said single hermetically packaged therapeutic dose inside said disposable dry powder inhaler device, said loading chamber comprising activation means having an activated position and a rest position, said activated position being provided to open said single hermetically packaged therapeutic dose and locking means provided to prevent extraction of the single hermetically packaged therapeutic dose once said activation means have been moved to said activated position.

In the present invention, each disposable dry powder inhaler device comprises a dispersion chamber in fluid connection with an inhalation channel and connected to a loading chamber for accommodating said single hermetically packaged therapeutic dose inside said disposable dry powder inhaler device, said loading chamber comprising activation means having an activated position and a rest position, said activated position being provided to open said single hermetically packaged therapeutic dose and locking means having an open position and a lock position provided to prevent extraction of the single hermetically packaged therapeutic dose, said activation means being provided to be able to be moved to said activated position only when said locking means are in said lock position.

Further, in the present invention, wherein each disposable dry powder inhaler device comprises a dispersion chamber in fluid connection with an inhalation channel and connected to a loading chamber for accommodating said single hermetically packaged therapeutic dose inside said disposable dry powder inhaler device, said loading chamber comprising activation means having an activated position and a rest position, said disposable dry powder inhaler device comprising closing means provided to isolate the outside environment from the inside of the disposable dry powder inhaler device, in particular by closing the inhalation channel.

In a preferred embodiment, said disposable dry powder inhaler device is ready to use and comprises a dispersion chamber in fluid connection with an inhalation channel and connected to a loading chamber comprising said single hermetically packaged therapeutic dose inside said disposable dry powder inhaler device.

In a particular embodiment of the present invention, said single hermetically packaged therapeutic dose comprises a therapeutic dose in the form of a dry and fine powder having a geometric particle size distribution (PSD) d₅₀ lower than or equal to 30 µm, preferably lower than 15 µm, preferably lower than or equal to 10 µm, preferably lower than or equal to 5 µm, and a geometric particle size distribution (PSD) d₉₀ lower than or equal to 60 µm, preferably lower than or equal to 30 µm, more preferably lower than or equal to 15 µm, preferably lower than or equal to 10 µm and more preferably lower than or equal to 7 µm, and optionally a volume mean diameter D[4,3] lower than or equal to 40 µm, preferably lower than or equal to 20 µm, more preferably lower than or equal to 15 µm, preferably lower than or equal to 10 µm, preferably lower than or equal to 6 µm.

According to the present invention, in a preferred embodiment, the single hermetically packaged therapeutic dose comprises a total powder weight content between 2 to 100 mg, preferably between 3 and 80 mg, more preferably between 4 and 60 mg, in particular between 5 and 50 mg, such as for example between 6 and 20, or between 7 and 15, like around 10 mg.

For example, if the anti-neoplastic agent is cisplatin, the single hermetically packaged therapeutic dose comprises a total powder weight content between 2 to 40 mg, preferably between 3 and 30 mg, more preferably between 4 and 20 mg, in particular between 5 and 15 mg, such as for example between 6 and 10 mg.

For example, if the anti-neoplastic agent is carboplatin, the single hermetically packaged therapeutic dose comprises a total powder weight content between 10 to 100 mg, preferably between 15 and 80 mg, more preferably between 20 and 60 mg, in particular between 30 and 50 mg.

In a further preferred embodiment according to the present invention, each single hermetically packaged therapeutic dose comprises between 0.1 and 90 mg, preferably between 1 mg and 40 mg of said anti-neoplastic agent.

For example, typical single hermetically packaged therapeutic dose comprises between 0.5 and 35 mg, preferably between 1 and 30 mg, in particular between 2 and 25 mg of said anti-neoplastic agent, such as preferred single hermetically packaged therapeutic dose included within the range of 3 to 15 mg of said anti-neoplastic agent such as within the range of 5 to 10 mg of said anti-neoplastic agent.

For example, if said anti-neoplastic agent is cisplatin, typical single hermetically packaged therapeutic dose comprises between 0.5 and 20 mg, preferably between 1 and 15 mg, more preferably between 2 and 10 mg, in particular, between 3 and 5 mg.

If said anti-neoplastic agent is carboplatin, typical single hermetically packaged therapeutic dose comprises between 2.5 and 80 mg, preferably between 5 and 60 mg, more preferably between 10 and 50 mg, in particular, between 15 and 25 mg.

Further, it is also possible that the typical single hermetically packaged therapeutic dose comprises a mixture of anti-neoplastic agents, each being possibly comprised at an amount of between 0.5 and 50 mg.

According to the present invention, each single hermetically packaged therapeutic dose comprises from 0,1 to 99.9 mg, preferably between 1 and 49 mg of said lipid matrix.

Preferably, according to the present invention, the single hermetically packaged therapeutic dose presents a weight ratio between said anti-neoplastic agent and said lipid matrix: anti-neoplastic agent / lipid matrix comprised between 10/90 and 90/10, preferably between 25/75 and 75/25, more preferably between 30/70 and 70/30, in particular between 40/60 and 60/40, such as for example around 50/50.

Advantageously, in the present invention, each single hermetically packaged therapeutic dose comprises from 0.01 to 5 mg, preferably between 0.03 mg and 1 mg of PEGylated excipient.

Other embodiments of the kit for an inhaled chemotherapy according to the present invention are mentioned in the appended claims

The present invention also relates the suitability of the use of a kit for inhaled chemotherapy in a polytherapy.

Advantageously, said polytherapy comprises one therapy chosen in the group consisting of systemic chemotherapy (including intravenous infusion and oral per os), hormonotherapy, targeted therapy, immunotherapy, tumor ablative surgery, ablative surgery for removing a part of or a full organ bearing a tumor, a curative surgery, a radiotherapy and their combination and at least one chemotherapy by inhalation as additional therapy.

Intravenous infusion chemotherapy can be composed of two anti-neoplastic agents (e.g. platinum doublets) or more.

Cumulative dose of cisplatin can be safely increased with inhaled chemotherapy. The current IV cumulative dose is 300 mg/m² within 4 to 6 cycles.

Said therapy comprises at least intravenous infusion or oral chemotherapy, an hormonotherapy, a targeted therapy or an immunotherapy provided to be administered for a series of cycles, each cycle comprising an administration period and an off-period, said at least chemotherapy by inhalation being provided to be administered in a period before said therapy and/or during said off-period of the therapy.

Preferably, in the use of the kit according to the present invention, said therapy comprises at least intravenous infusion or oral chemotherapy or an immunotherapy provided to be administered for a series of cycles, each cycle comprising an administration period and an off-period, said at least one chemotherapy by inhalation being provided to be administered in a period before said therapy and/or during said off-period of the therapy.

The therapy can be generally administered orally or parenterally. Further, said at least one chemotherapy by inhalation can comprise a series of chemotherapy by inhalation which can be administered sequentially or together such as in a mixture. Sequentially means that one chemotherapy can be administered immediately after another chemotherapy or later, according to the same dosage regimen or according to another dosage regimen.

Preferably, said administration period extends over less than one day, preferably over a period of time between 15 minutes to 6 hours, preferably between 30 minutes and 4 hours, such as between 1 and 3 hours and where said off-period extends over a period of time from 1 to 5 weeks, preferably from 2 to 4 weeks, more preferably between 2,5 and 3,5 weeks and particularly over about 3 weeks ± 2 days.

Also preferred is that said at least one chemotherapy by inhalation is provided to be administered at a rate of at least 1 single hermetically packaged therapeutic dose of anti-neoplastic agent /week of period before said therapy and/or during said off-period, preferably of at least 3 single hermetically packaged therapeutic doses of anti-neoplastic agent /week of period before said therapy and/or during said off-period, more preferably of at least 5 single hermetically packaged therapeutic doses of anti-neoplastic agent /week of period before said therapy and/or during said off-period, possibly 7 single packaged therapeutic doses of anti-neoplastic agent /week of period before said therapy and/or during said off-period for a daily dosage regimen, optionally in an equally spread manner over said period before said therapy and/or during said off-period.

Further preferred is that said at least one chemotherapy by inhalation is provided to be administered at a rate of one or more single hermetically packaged therapeutic dose(s) of anti-neoplastic agent during the same administration event, with the same anti-neoplastic agent or with different anti-neoplastic agents. When more than one single hermetically packaged therapeutic doses (with the same anti-neoplastic agent or with different anti-neoplastic agents) are administered for one administration event, the single hermetically packaged doses of each anti-neoplastic agent can be administered sequentially (together) or spaced by a predetermined period of time, such as one single hermetically packaged dose each 2 hours, 3 single hermetically packaged doses forming one administration event.

Said at least one chemotherapy by inhalation may be administered at a rate of at least 1 administration event/week of period before said therapy and/or during said off-period, preferably of at least 3 administration event/week of period before said therapy and/or during said off-period, more preferably of at least 5 administration event/week of period before said therapy and/or during said off-period, possibly 7 administration event/week of period before said therapy and/or during said off-period for a daily dosage regimen, optionally in an equally spread manner over said period before said therapy and/or during said off-period.

If said off-period is one week, the chemotherapy by inhalation can be administered at a rate of 1, 2, 3, 4, 5 or even 7 single hermetically packaged therapeutic dose of anti-neoplastic agent. Preferably, the chemotherapy by inhalation can be administered at a rate of 2 to 5, or 3 to 5 single hermetically packaged therapeutic doses of anti-neoplastic agent by said week off-period.

If the period before said therapy is one week, the chemotherapy by inhalation can be administered at a rate of 1, 2, 3, 4, 5 or even 7 single hermetically packaged therapeutic doses of anti-neoplastic agent. Preferably, the chemotherapy by inhalation can be administered at a rate of 2 to 5 or 3 to 5 single hermetically packaged therapeutic doses of anti-neoplastic agent by said week before the therapy.

If said off-period is three weeks, the at least one chemotherapy by inhalation can be administered at a rate of one single hermetically packaged therapeutic dose of anti-neoplastic agent each day, meaning between 15 and 21 doses of anti-neoplastic agent for the off-period of about 3 weeks. Optionally, the at least one chemotherapy by inhalation can be administered at a rate of one single hermetically packaged therapeutic dose of anti-neoplastic agent each two days, meaning between 9 and 12 doses of anti-neoplastic agent for the off-period of about 3 weeks. Alternatively, the at least one chemotherapy by inhalation can be administered at a rate of one single hermetically packaged therapeutic dose of anti-neoplastic agent each three days, meaning between 6 and 9 doses of anti-neoplastic agent for the off-period of about 3 weeks.

If the period before said therapy is for example 2 weeks, the at least one chemotherapy by inhalation can be administered at a rate of 1, 2, 3, 4, 5 or even 7 single hermetically packaged therapeutic doses of anti-neoplastic agent each week. Preferably, the at least one chemotherapy by inhalation can be administered at a rate of 2 to 5 or 3 to 5 single hermetically packaged therapeutic doses of anti-neoplastic agent each week by said 2 weeks before the therapy.

A typical chemotherapy scheme can be for example a sequence of 2 weeks before the therapy, a series of cycles of one administration followed by 3 weeks off-period. In this case, the at least one chemotherapy by inhalation can be administered during the 2 weeks before the therapy at a frequency (such as one single hermetically packaged therapeutic dose of anti-neoplastic agent each day or two days), not administered on the date of the administration of the therapy and again administered during the off-period, for example starting immediately the same day, after some hours, or the day after or even 2 or 3 days after the therapy, and then over the remaining time of the off-period at a frequency of one single hermetically packaged therapeutic dose of anti-neoplastic agent daily.

Similarly, if said off-period is four weeks, the at least one chemotherapy by inhalation can be administered at a rate of one single hermetically packaged therapeutic dose of anti-neoplastic agent each day, meaning between 20 and 28 doses of anti-neoplastic agent for the off-period of about 4 weeks. Optionally, the at least one chemotherapy by inhalation can be administered at a rate of one single hermetically packaged therapeutic dose of anti-neoplastic agent each two days, meaning between 12 and 16 doses of anti-neoplastic agent for the off-period of about 4 weeks. Alternatively, the at least one chemotherapy by inhalation can be administered at a rate of one single hermetically packaged therapeutic dose of anti-neoplastic agent each three days, meaning between 8 and 12 doses of anti-neoplastic agent for the off-period of about 4 weeks.

According to the present invention, one particular embodiment of the kit for an inhaled chemotherapy is suitable for use in a polytherapy further comprising at least tumor ablative surgery, ablative surgery for removing a part of or a full organ bearing a tumor, a curative surgery, or a radiotherapy followed by a recovery period, said at least one chemotherapy by inhalation being provided to be administered in a period before, during or after said further therapy, such as for example during or after said recovery period, during which typically no orally or parenterally chemotherapy is administered.

Advantageously, in the kit for an inhaled chemotherapy according to the present invention suitable for use in polytherapy, said at least one chemotherapy by inhalation is provided to be administered at a rate of at least 1 single hermetically packaged therapeutic dose of anti-neoplastic agent /week of in a period before, during or after said therapy, preferably of at least 3 single hermetically packaged therapeutic doses of anti-neoplastic agent/week of in a period before, during or after said therapy, more preferably of at least 5 single hermetically packaged therapeutic doses of anti-neoplastic agent/week of in a period before, during or after said therapy, possibly 7 single packaged therapeutic doses of anti-neoplastic agent/week of in a period before, during or after said therapy for a daily dosage regimen, optionally in an equally spread manner over said in a period before, during or after said therapy.

The kit for a inhaled chemotherapy of the present invention offers a treatment against lung cancer comprising administering at least one inhaled chemotherapy in a polytherapy comprising at least one therapy chosen in the group consisting of intravenous infusion or oral chemotherapy, hormonotherapy, targeted therapy, immunotherapy, tumor ablative surgery, ablative surgery for removing a part of or a full organ bearing a tumor, a curative surgery, a radiotherapy and their combination, said at least one inhaled chemotherapy being an additional therapy.

Advantageously, said therapy comprises at least intravenous infusion or oral chemotherapy, hormonotherapy, targeted therapy or an immunotherapy provided to be administered for a series of cycles, each cycle comprising an administration period and an off-period, said at least one chemotherapy by inhalation being provided to be administered before said therapy and/or during said off-period of the therapy.

Advantageously, said therapy comprises at least intravenous infusion or oral chemotherapy or an immunotherapy provided to be administered fora series of cycles, each cycle comprising an administration period and an off-period, said at least one chemotherapy by inhalation being provided to be administered before said therapy and/or during said off-period of the therapy

Preferably, said administration period extends over less than one day, preferably over a period of time between 15 minutes to 6 hours, preferably between 30 minutes and 4 hours, such as between 1 and 3 hours, and where said off- period extends over a period of time from 2 to 5 weeks, preferably from 2 to 4 weeks, more preferably between 2,5 and 3,5 weeks and particularly over about 3 weeks + 2 days.

More particularly, the at least one chemotherapy by inhalation is provided to be administered at a rate of at least 1 single hermetically packaged therapeutic dose of anti-neoplastic agent/week of period before said therapy and/or during said off-period, preferably of at least 3 single hermetically packaged therapeutic doses of anti-neoplastic agent/week of period before said therapy and/or during said off-period, more preferably of at least 5 single hermetically packaged therapeutic doses of anti-neoplastic agent/week of period before said therapy and/or during said off-period, possibly 7 single packaged therapeutic doses of anti-neoplastic agent/week of period before said therapy and/or during said off-period for a daily dosage regimen, optionally in an equally spread manner over said period before said therapy and/or during said off-period.

In a variant, said polytherapy comprises at least tumor ablative surgery, ablative surgery for removing a part of or a full organ bearing a tumor, a curative surgery, or a radiotherapy followed by a recovery period, said at least one chemotherapy by inhalation being provided to be administered on a period before, during or after said therapy.

In this variant, said at least one inhaled chemotherapy is administered at a rate of at least 1 single hermetically packaged therapeutic dose of anti-neoplastic agent/week of period before, during or after said therapy, preferably of at least 3 single hermetically packaged therapeutic doses of anti-neoplastic agent/week of period before, during or after said therapy, more preferably of at least 5 single hermetically packaged therapeutic doses of anti-neoplastic agent/week of period before, during or after said therapy, possibly 7 single packaged therapeutic doses of anti-neoplastic agent/week of period before, during or after said therapy for a daily dosage regimen, optionally in an equally spread manner over said period before, during or after said therapy

In particular, said lung cancer is any lung tumor, such as pulmonary metastases, for example osteosarcoma metastases, a small cell lung cancer or a non-small cell lung cancer.

Other characteristics and advantages of the present invention will be derived from the non-limitative following description, and by making reference to the drawings and the examples.

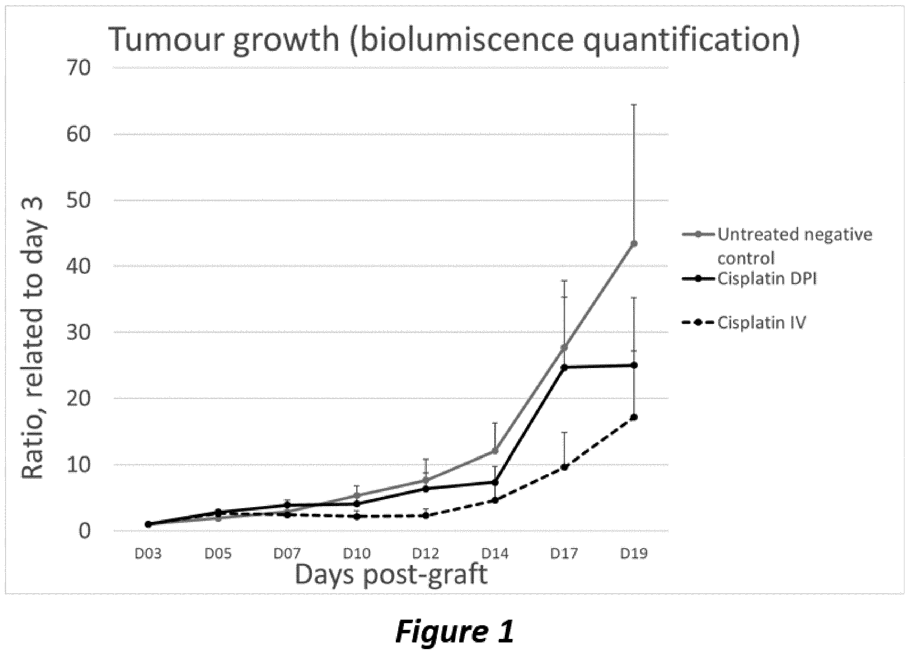

In the drawings, figure 1 shows the tumor growth for the two cisplatin-treated groups, compared with the untreated control group of example 3. Data are presented as the mean ratio of bioluminescence signals related to day 3 post graft ± SEM.

Figure 2 shows the survival rates (p<0.05, Log-rank Test) for the two cisplatin-treated groups, compared with the untreated control group of example 3.

Figure 3 shows the tumor growth in the chest and in the lungs for cisplatin IV compared with cisplatin DPI from example 3. Data are presented as mean bioluminescence signal ± SEM.

Figure 4 shows the tumor growth for the two cisplatin-treated groups, compared with the untreated control group of example 4. Data are presented as the mean ratio of bioluminescence signals related to day 3 post graft ± SEM.

Figure 5 shows the survival rates (p<0.05, Log-rank Test) for the two cisplatin-treated groups, compared with untreated the untreated control group of example 4.

Figure 6 shows the results of bioluminescence assays of tumor growth for example 5.

Figure 7 shows the survival rates (p<0.05, Log-rank Test) for the groups of example 5.

Figure 8 shows the results of bioluminescence assays of tumor growth for example 6.

Figure 9 presents the pictures obtained in bioluminescence at days 7 and 38 for the 2 mice remaining alive at day 38, one mouse in the anti-PD1 monotherapy group and one in the combination group of example 6.

Figure 10 shows the results of bioluminescence assays of tumor growth for example 9.

Figure 11 shows the survival rates (p<0.05, Log-rank Test) for the groups of example 9.

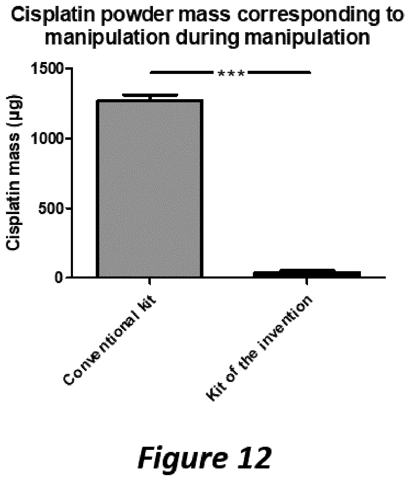

Figure 12 shows the cisplatin powder fraction responsible for contamination from example 10.

In the drawings, the same reference numbers have been allocated to the same or analog element

### Examples.-

### Example 1.- preparation of cisplatin dry powder formulation n°1

Briefly, raw cisplatin microcrystals from bulk powder (Shanghai Jinhe Bio-technology Co., Ltd., Shanghai, PRC) were first suspended in 50 mL isopropanol to reach a 5% w/v suspension and reduced in size by high-speed (10 min at 24 000 rpm) (X620 motor and a T10 dispersing shaft, Ingenieurbüro CAT M. Zipperer GmbH, Staufen, Germany) and high-pressure homogenization (EmulsiFlex-C5 high-pressure homogenizer, Avestin Inc., Ottawa, Canada) at 5 000 psi then 10 000 psi over 10 pre-milling cycles each, then at 20 000 psi over 20 milling cycles. A heat exchanger was connected to the homogenizing valve and maintained at -15°C using an F32-MA cooling circulator (Julabo GmbH, Seelbach, Germany). An aliquot was removed from the suspension at the end of the process to measure the particle size distribution (PSD) of cisplatin microcrystals by laser diffraction (see below).

Then, tristearin (Tokyo Chemical Company, Tokyo, Japan) and TPGS (Sigma-Aldrich, St-Louis, USA) solubilized in heated isopropanol were added to the microcrystal suspension to obtain a final concentration of 1.0% w/v of cisplatin and 1.0% w/v of tristearin/TPGS (99:1 w/w) mixture and spray dried with a Mini-Spray Dryer B-290 (Büchi Labortechnik AG, Flawil, Switzerland) to obtain a DPI formulation for human use.

The operating parameters used during spray-drying were as follows: feed rate 3.0 g/min, inlet temperature 70°C, 0.7 mm nozzle, 1.5 mm nozzle-cap, compressed air at 800 L/min and drying air flow 35 m³/h. The apparatus was equipped with a B-296 dehumidifier (Büchi Labortechnik AG) to maintain the relative humidity at 50% HR during spray-drying.

The geometrical PSD of bulk cisplatin, cisplatin microparticles from the size-reduction process, and cisplatin dry powder formulation, was measured as suspended and individualized particles. This was done using a Mastersizer 3000 laser diffractometer (Malvern Instruments Ltd., Worcestershire, UK) connected to a Hydro MV dispenser equipped with a 40 W ultrasonic probe (Malvern Instruments Ltd.). Measurements of bulk cisplatin and aliquots of cisplatin microparticles from the size-reduction process were performed in cisplatin-saturated isopropanol. The cisplatin dry powder formulation was previously dispersed, vortexed and measured in cisplatin- saturated NaCl 0.9% aqueous solution containing 0.1% w/v Poloxamer 407 (BASF, Ludwigshafen, Germany). The PSD was expressed as the volume median diameter d₅₀ (for which 50% of particles lie under the expressed diameter) and the volume mean diameter D[4,3]. The results are presented in table 1.

**Table 1.- Volume median diameter d₅₀ (for which 50% of particles lie under the expressed diameter), and the volume mean diameter D[4,3] of raw cisplatin, cisplatin microparticles from the size-reduction process, and cisplatin dry powder formulation according to Example 1. Data are expressed as the mean ± SD, n = 3.**

| | D₅₀ (µm) | D[4,3] (µm) |
|---|---|---|
| Raw cisplatin | 16.5 ± 3.6 | 18.9 ± 4.3 |
| Cisplatin microparticles | 0.89 ± 0.01 | 1.0 ± 0.1 |
| Cisplatin dry powder formulation according to Example 1 | 4.2 ± 0.2 | 4.4 ± 0.1 |

### Example 2.- Preparation of cisplatin dry powder formulation n°2

Cisplatin (Umicore, Hanau-Wolfgang, Germany) was first suspended in 50 mL ethanol to reach a concentration of 5% w/v and reduced in size by high-speed (10 min at 24 000 rpm) (X620 motor and a T10 dispersing shaft, Ingenieurbüro CAT M. Zipperer GmbH, Staufen, Germany) and high-pressure homogenization using an EmulsiFlex-C3 high-pressure homogenizer (Avestin Inc., Ottawa, Canada) for 40 cycles at 20,000 PSI. These cycles were conducted by recirculating the processed suspension directly into the sample tank (closed loop). Because HPH causes a sample temperature increase, all operations were carried out using a heat exchanger placed ahead of the homogenizing valve, with the sample temperature maintained at 15 ± 1 °C.

An aliquot was removed from the suspension at the end of the process to measure the PSD of cisplatin microcrystals by laser diffraction (see below).

Then, castor oil hydrogenated (BASF, Ludwigshafen, Germany) and TPGS (Sigma-Aldrich, St-Louis, USA) solubilized in heated isopropanol were added to the microcrystal suspension to obtain a final concentration of 2.0% w/v of cisplatin and 2.0% w/v of castor oil hydrogenated/TPGS (99:1 w/w) mixture and spray dried with a Mini-Spray Dryer B-290 (Büchi Labortechnik AG, Flawil, Switzerland) to obtain a DPI formulation for human use. The operating parameters used during spray-drying were as follows: feed rate 3.0 g/min, inlet temperature 70°C, 0.7 mm nozzle, 1.5 mm nozzle-cap, compressed air at 800 L/min and drying air flow 35 m³/h. The apparatus was equipped with a B-296 dehumidifier (Büchi Labortechnik AG) to maintain the relative humidity at 50% HR during spray-drying.

The geometrical PSD of bulk cisplatin, cisplatin microparticles from the size-reduction process, and cisplatin dry powder formulation, was measured as suspended and individualized particles as described in the Example 1 and are presented in table 2.

**Table 2.- Volume median diameter d₅₀ (for which 50% of particles lie under the expressed diameter) and the volume mean diameter D[4,3] of raw cisplatin, cisplatin microparticles from the size-reduction process, and cisplatin dry powder formulation according to Example 2. Data are expressed as the mean ± SD, n = 3.**

| | D₅₀ (µm) | D[4,3] (µm) |
|---|---|---|
| Raw cisplatin | 6.3 ± 0.1 | 7.1 ± 0.2 |
| Cisplatin microparticles | 0.68 ± 0.01 | 0.94 ± 0.01 |
| Cisplatin dry powder formulation according to Example 2 | 2.240 ± 0.001 | 2.87 ± 0.01 |

### Example 3.- Cisplatin administration to mice model 1: Cisplatin as a monotherapy

All the animal studies were approved by ULB's ethical committee from the Faculty of Medicine (protocol 424N and 585N) and Center for Microscopy and Molecular Imaging (protocol CMMI 2017 01).

### Lung cancer mice model.

The orthotopic intrapulmonary implantation procedure was performed using an adaption of the protocol described previously (Wauthoz et al., Nanomedicine 2015). Briefly, under anesthesia, 6 to 7-week-old BALB/c mice were grafted with 1.4 million M109-HiFR-*LUC*2 cells in 20 µL of a 50:50 v/v mix of folate-free RPMI/Matrigel^{®} injected intercostally after incision of the skin and dissection of subcutaneous muscles. The different tissue incisions were stitched with a 3-0 silk (Ethicon^{®}, St-Stevens-Woluwe, Belgium). The mice were closely observed until complete recovery. The therapeutic response of the M109 model to cisplatin was evaluated by *in vivo* optical imaging (bioluminescence) and survival analysis. Bioluminescence is directly proportional to the number of living M109-HiFR-LUC2 tumor cells. Bioluminescence was performed up to day 19 post tumor cell engraftment using a PhotonImager RT (Biospace Lab, France) using the conventional supplier's protocol. Mice were weighted three times a week and sacrificed if their weight loss exceeded 20% from the beginning of the experiment or 15% from the last measurement or if they were suffocating. In these cases, the mouse death was notified as occurred the day after.

Three groups of 6-8 mice were compared: (i) untreated negative control, (ii) mice injected intravenously (IV) with 1.5 mg/kg cisplatin in a one-week cycle (one administration per cycle in weeks 1 and 3, week 2 is a week-off), (iii) mice treated with cisplatin DPI prepared in example 1 at a dose of 0.5 mg/kg cisplatin in a one-week cycle (three administrations per cycle in weeks 1 and 3, week 2 is a week-off) using an endotracheal device adapted to mice (Dry Powder Insufflator^{™} model DP4-M^{®}, Penn-Century, Philadelphia, USA) and using the protocol described by Levet et al (Levet et al., Int J Pharm 2017). Treatments were administered from day 3 post tumor cell engraftment.

As it can be seen in figure 1, a decrease in tumor growth for the two cisplatin-treated groups, compared with the untreated control group was observed, but the differences were not statistically significant (p>0.05, 1-way ANOVA). Data are presented as the mean ratio of bioluminescence signals related to day 3 post graft ± SEM.

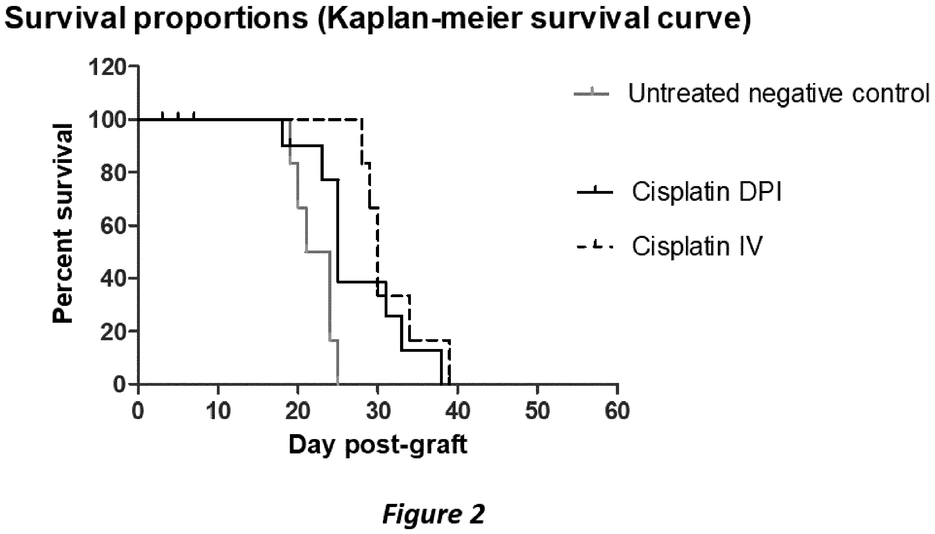

As it can be seen on figure 2, a significant increase in survival rates (p<0.05, Log-rank Test) for the two cisplatin-treated groups, compared with the untreated control group was observed. No significant differences between cisplatin DPI and cisplatin IV is observed (p>0.05, Log-rank Test).

An additional experiment was performed evaluating cisplatin DPI according to example 1 vs cisplatin IV on the same lung cancer mice model.

The mice were euthanized on day 19 post tumor cell engraftment to perform an *ex vivo* bioluminescence analysis to analyze tumor localization in the resected lungs (as considered as the primary lung tumors) vs in the chest cavity (as considered as loco-regional metastases).

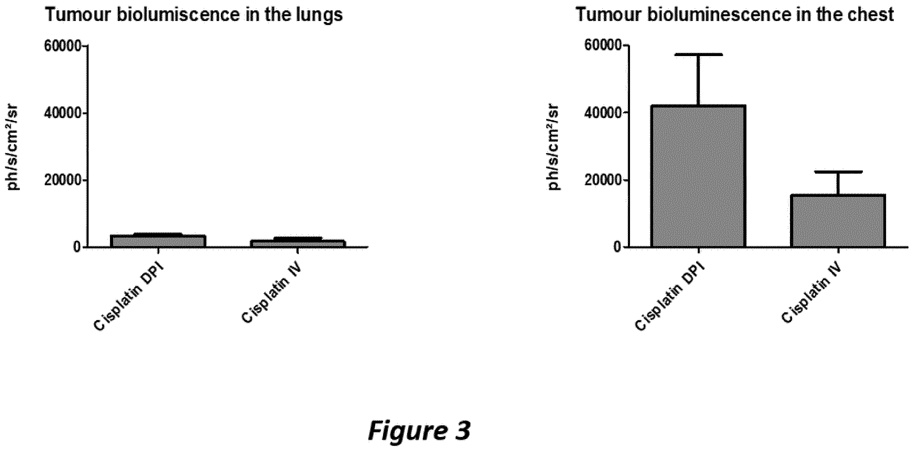

As it can be seen in figure 3, a decrease of tumor growth in the chest for cisplatin IV compared with cisplatin DPI was observed but the tumor growth in the lungs was similar. Data are presented as mean bioluminescence signal ± SEM.

### Example 4.- Combination of cisplatin DPI with systemic cisplatin administration to mice model

The protocol described in example 3 has been also performed with further groups. Three groups of 6-10 mice were compared:
(i) untreated negative control,
(ii) mice injected IV with 1.5 mg/kg cisplatin in a one-week cycle for two cycles (one administration per cycle in weeks 1 and 3, week 2 is a week-off),
(iii) mice treated with a combination of cisplatin DPI according to example 1 at a dose of 0.5 mg/kg cisplatin and cisplatin IV at 1.5 mg/kg in a one-week cycle (three administrations per cycle in weeks 1 and 3, week 2 is a week-off).

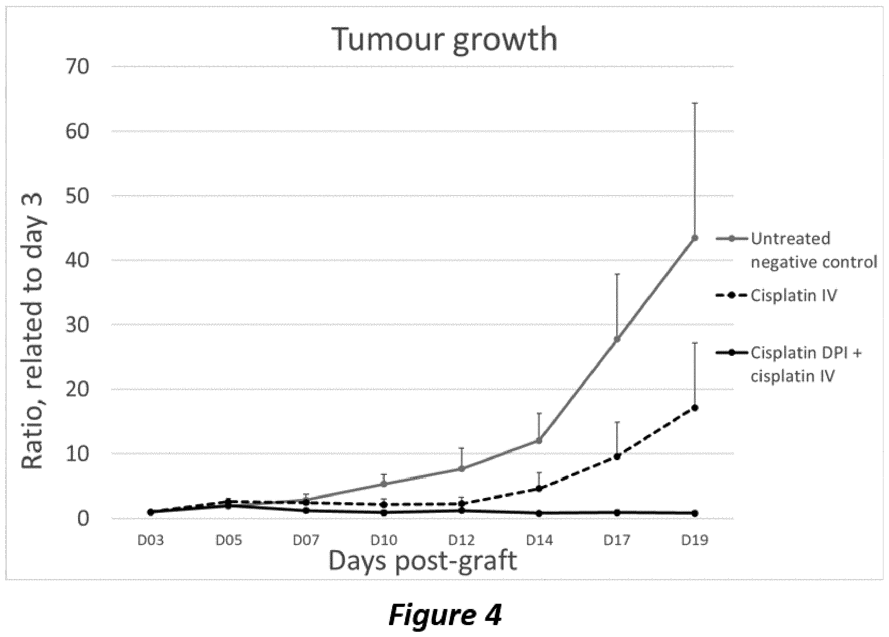

As it can be seen in figure 4, a decrease in tumor growth for the two cisplatin-treated groups compared with the untreated control group was observed. This decrease was significant for the combination (iii) (p<0.05, 1-way ANOVA) but not for the cisplatin IV (ii) (p>0.05, 1-way ANOVA). Data are presented as the mean ratio of bioluminescence signals related to day 3 post graft ± SEM.

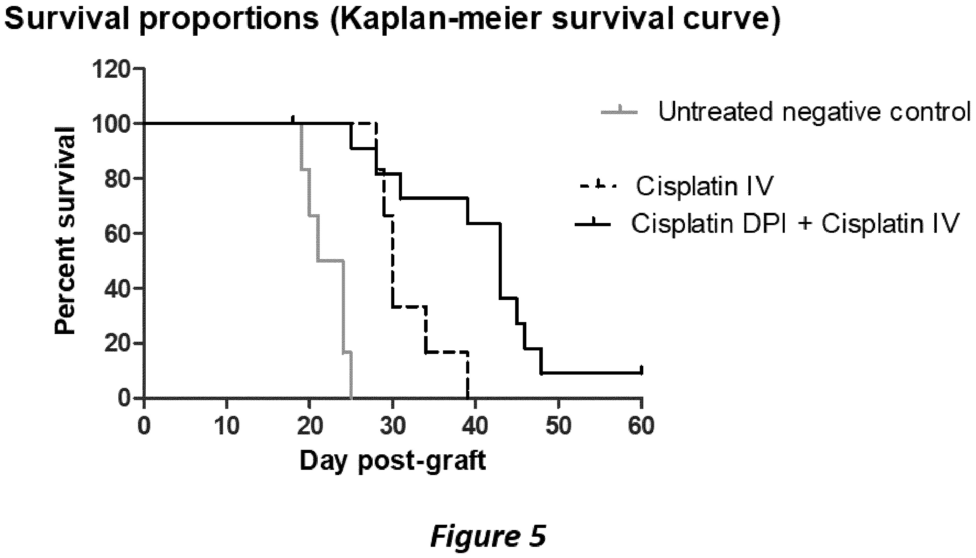

As it can be seen in figure 5, a significant increase in survival rates (p<0.05, Log-rank Test) for the two cisplatin-treated groups compared with the untreated control group was observed. Significant increase in survival rates (p<0.05, Log-rank Test) for the combination compared with cisplatin IV was also observed.

### Example 5.- Combination of cisplatin DPI with systemic cisplatin and paclitaxel as systemic platinum doublets (Pt doublets) administration to mice model

The aim of the example is to demonstrate an additive/synergistic effect between cisplatin Dry Powder Inhalation (CIS DPI) and conventional systemic chemotherapy, i.e. platinum-based doublets (Pt doublets), widely used in stage III and IV non-small cell lung cancer (NSCLC) patients who are currently treated by chemotherapy as first, second and following line treatments.

Two main parameters were carefully followed during the study:
- The tumour growth over time by bioluminescence (in vivo optical imaging, the bioluminescence signal is directly proportional to the number of living M109 tumour cells *in vivo).* Bioluminescence analysis was performed two times a week, from day 3 (randomization) to day 38.
- Survival analysis, including Kaplan Meier survival curve and median survival (corresponding to the time where 50% mice died of their lung tumours and/or metastases).

The protocol described in example 3 has been reproduced, except that survival rates were also determined (no euthanasia on day 19 and no ex vivo bioluminescence analysis), the treatment started on day 6 according to the scheme table 3 below and that three groups of 4-15 mice were compared:
(i) untreated negative controls which were injected iv with saline,
(ii) mice injected IV with a platinum doublet composed of 1.5 mg/kg cisplatin and 10 mg/kg paclitaxel in a one-week cycle (a single IV injection per cycle) for 2 consecutive cycles,
(iii) mice treated with a combination of CIS DPI at a dose of 0.5 mg/kg cisplatin (from example 2) and the platinum doublet in a one-week cycle (3 CIS DPI administrations and a single Pt doublets IV injection per cycle) for 2 consecutive cycles.

**Table 3.- Dose regimen and scheme of administration followed in Example 5**

| **Groups:** | **i. Negative control** | **ii. Platinum doublet iv** | **iii. Platinum doublet iv + CIS DPI** |
|---|---|---|---|
| **Day 0** | M109 cell engraftment | | |
| **Day 3** | Randomization | | |
| **Day 6** | Saline iv | 1.5 mg/kg cisplatin iv and 10 mg/kg paclitaxel iv | 1.5 mg/kg cisplatin iv and 10 mg/kg paclitaxel iv |
| **Day 7** | Off-period | Off-period | 0.5 mg/kg CIS DPI |
| **Day 8** | | | Off-period |
| **Day 9** | | | 0.5 mg/kg CIS DPI |
| **Day 10** | | | Off-period |
| **Day 11** | | | 0.5 mg/kg CIS DPI |
| **Day 12** | | | Off-period |
| **Day 13** | Saline iv | 1.5 mg/kg cisplatin iv and 10 mg/kg paclitaxel iv | 1.5 mg/kg cisplatin iv and 10 mg/kg paclitaxel iv |
| **Day 14** | Off-period | Off-period | 0.5 mg/kg CIS DPI |
| **Day 15** | | | Off-period |
| **Day 16** | | | 0.5 mg/kg CIS DPI |
| **Day 17** | | | Off-period |
| **Day 18** | | | 0.5 mg/kg CIS DPI |
| **Day 19** | | | Off-period |

The mice that died during the endotracheal administration procedure are not considered in the analysis - 7/15 died during the procedures in the combination group.

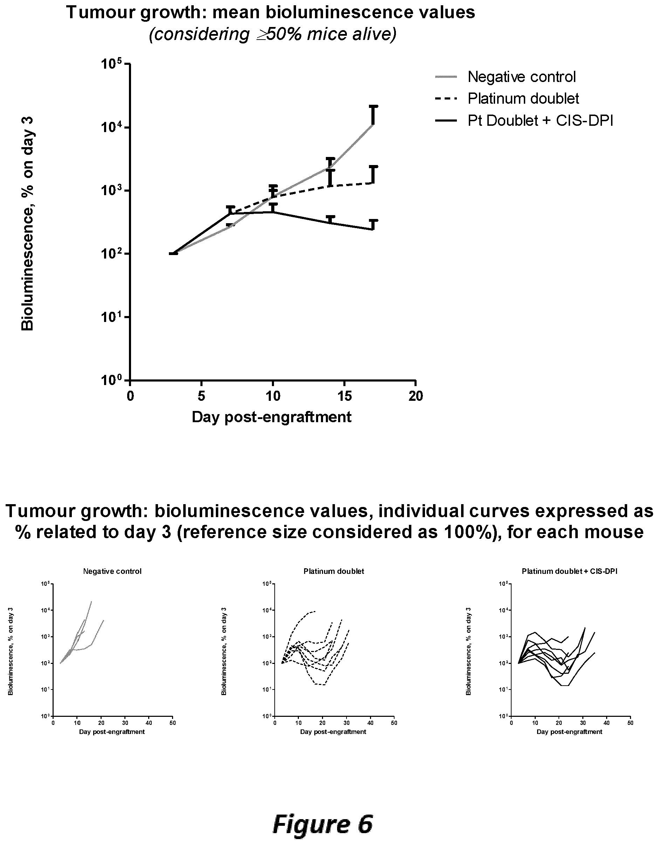

As it can be seen in figure 6, the addition of CIS-DPI to the platinum doublet allowed a better control of tumor growth during treatment (until day 18, which corresponded to the last administered dose), with a large majority of responders (see individual curves), which was more discernible than with the platinum doublet:
- Response to the platinum doublet:
   ∘ Most of the mice (7/8, 88%) responded to the platinum doublets. However, this was a temporary response, as observed with the re-growth of the tumor from day 21.
   o Reduction of the tumor cells (temporary bioluminescence values < 100% - tumor size under the day 3 reference, i.e. before treatment) was observed in 4/8 (50%) mice.
- Response to the platinum doublet + CIS-DPI
   ∘ All the mice (8/8, 100%) responded to the combination, with 6/8 (75%) mice having reduction of the tumor cells compared to day 3 reference (values < 100%).

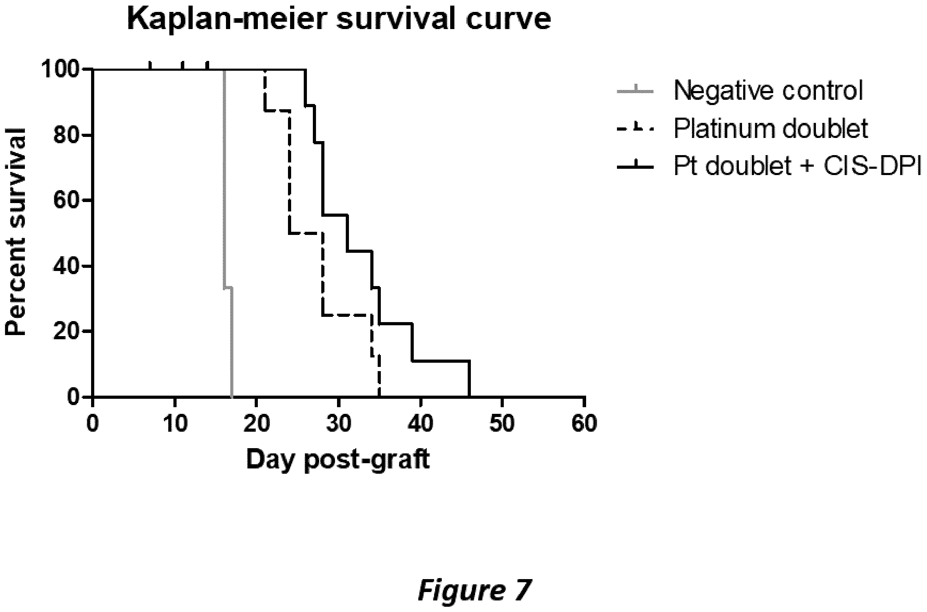

As it can be seen in figure 7 and in table 4, the addition of CIS-DPI to the platinum doublet tended to prolong the survival rates (p=0.10, log-rank test), with a median survival increase of 20% compared to the platinum doublet (31 vs 26 days, respectively).

**Table 4.- Survival rate at different timepoints**

| **Group** | **Mice alive (%) at day 24:** | **Mice alive (%) at day 28:** | **Mice alive (%) at day 35:** |
|---|---|---|---|
| Pt doublet + CIS-DPI | 8/8 (100%) | 4/8 (50%) | 2/8 (25%) |
| Pt doublet | 5/8 (62%) | 2/8 (25%) | 0/8 (0%) |
| negative control | 0/4 (0%) | 0/4 (0%) | 0/4 (0%) |

### Example 6. Combination of cisplatin DPI with systemic immunotherapy

The aim of this example is to demonstrate a synergistic effect between CIS DPI and immune checkpoint inhibitors as monotherapy, i.e. not combined with iv chemotherapy, which is the current standard of care in -30% stage IV NSCLC (PD-L1 biomarker expression > 50%).

Two main parameters were carefully followed during the study:
- The tumour growth over time by bioluminescence (in vivo optical imaging, the bioluminescence signal is directly proportional to the number of living M109 tumour cells *in vivo*). Bioluminescence analysis was performed two times a week, from day 3 (randomization) to day 38.
- Survival analysis, including Kaplan Meier survival curve and median survival (corresponding to the time where 50% mice died of their lung tumours or metastases.

The protocol described in example 3 has been reproduced, except that survival rates were also determined (no euthanasia on day 19 and no ex vivo bioluminescence analysis), the treatment started on day 3 according to the scheme table 5 below and three groups of 8-16 mice were compared:
(i) Negative controls, i.e. saline iv injection + Anti-βGAL rat IgG2a intraperitoneal (ip) injection (isotype control of anti-PD1, BioXCell, West Lebanon, NH, USA) + DPI vehicle (i.e. the powder without cisplatin, only the excipients),
(ii) mice injected ip with an anti-PD1 antibody (RMP 1-14, BioXCell, West Lebanon, NH, USA) in a one-week cycle for one cycle according to the scheme below,
(iii) mice treated with a combination of cisplatin DPI at a dose of 0.5 mg/kg cisplatin (from example 2) and the anti-PD1 antibody IP (RMP 1-14, BioXCell, West Lebanon, NH, USA) in a one-week cycle for one cycle according to the scheme below.

**Table 5.- Dose regimen and scheme of administration followed in Example 6**

| **Groups:** | **i. Negative control** | **ii. anti-PD1 ip** | **iii. anti-PD1 ip + CIS DPI** |
|---|---|---|---|
| **Day 0** | M109 cell engraftment | | |
| **Day 3** | - Randomization - Vehicle DPI | Randomization | - Randomization - 0.5 mg/kg CIS DPI |
| **Day 5** | Vehicle DPI | | 0.5 mg/kg CIS DPI |
| **Day 7** | - Isotype 5 mg/kg - Vehicle DPI | Anti-PD1 5 mg/kg | - Anti-PD1 5 mg/kg - 0.5 mg/kg CIS DPI |
| **Day 8** | Isotype 5 mg/kg | Anti-PD1 5 mg/kg | Anti-PD1 5 mg/kg |
| **Day 9** | - Isotype 5 mg/kg - Vehicle DPI | Anti-PD1 5 mg/kg | - Anti-PD1 5 mg/kg - 0.5 mg/kg CIS DPI |
| **Day 10** | Isotype 5 mg/kg | Anti-PD1 5 mg/kg | Anti-PD1 5 mg/kg |

The mice that died during the endotracheal administration procedure were not considered in the analysis - 9/16 of negative controls and 4/11 of cisplatin DPI + anti-PD1 died during the procedures.

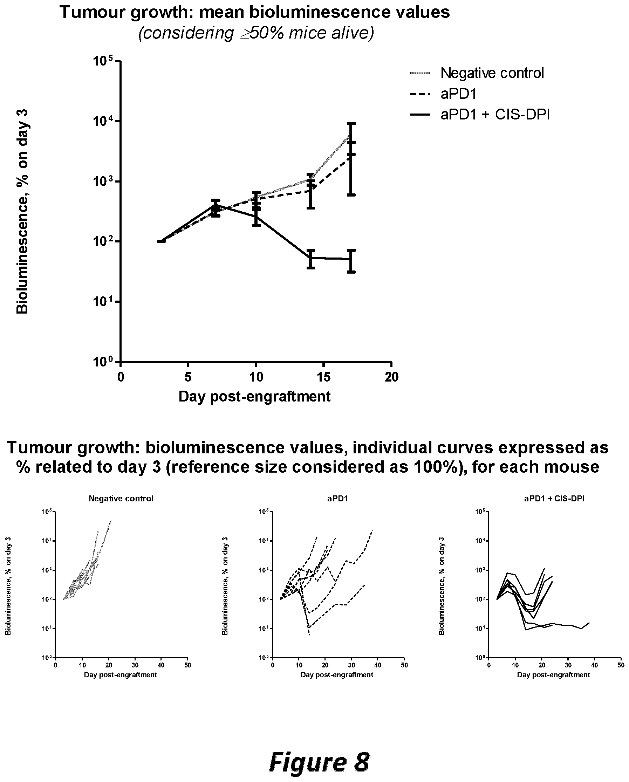

The data presented in Figure 8 demonstrated a potential synergy of cisplatin DPI with immunotherapy. While no reduction of tumor size was observed after 2 administrations of cisplatin DPI without anti-PD1 (i.e. at days 3 and 5), a sharp reduction was observed from day 10 once aPD1 was administrated but only in the combination group, with mean values below 100% at days 14 and 17, indicating a reduction of tumour cell number compared to day 3 (reference tumour size 100%). In contrast, tumour growth of anti-PD1-treated mice followed the negative controls'.
- Negative controls: not controlled and continuous tumour growth, with more than 500-fold increase of tumour cell number between day 3 and 21.
- Response to the immunotherapy: 3/8 mice (37.5%) responded to anti-PD1 monotherapy but no long responders observed. For these 3 responders, re-growth of the tumour after treatment, from day 14.
- Response to the combination of cisplatin DPI with immunotherapy:
   o All the mice (7/7, 100%) responded to the combination with an initial tumor growth up to -day 10 followed by a sharp decrease of the tumor size and a control of tumor growth up to day 17 observed in all the mice.
   ∘ Indubitable additive effect of cisplatin DPI on anti-PD1 therapy up to day 17, i.e. during the time the treatment is active.

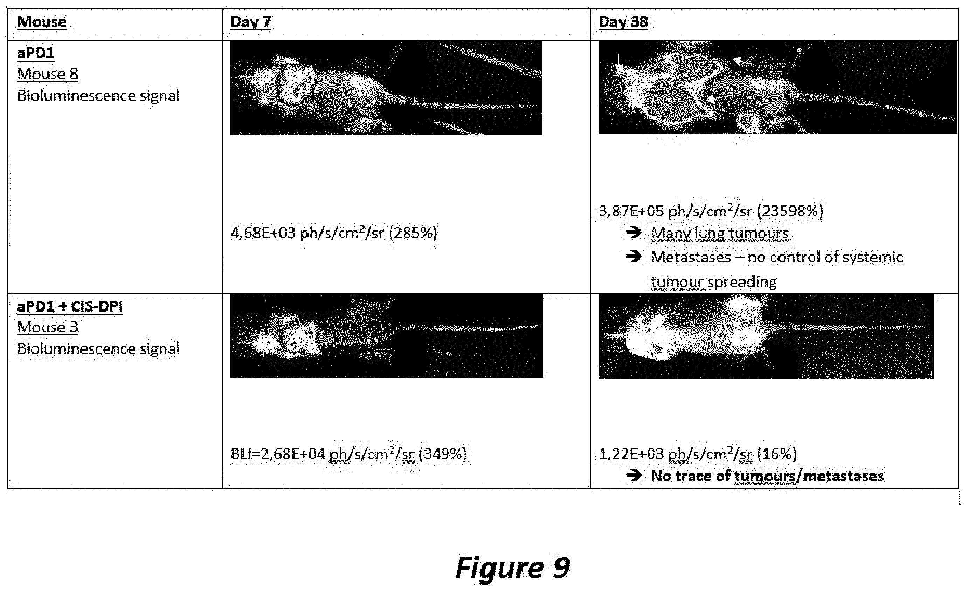

Moreover, in the combination group, 1/7 (14%) long responder was observed with no trace of any tumor, neither primary lung tumor nor locoregional nor systemic metastases (Fig 9). In contrast with the anti-PD1 monotherapy group, no long responder was observed (Fig 9).

As presented in table 6, the addition of cisplatin DPI to anti-PD1 immunotherapy tend to prolong survival rates, in particular before day 27, with median survivals of 23 days and 27 days for antiPD1 and anti-PD1 + cisplatin DPI, respectively.

**Table 6.- Survival rate at different timepoints**

| **Group** | **Mice alive (%) at day 17:** | **Mice alive (%) at day 21:** | **Mice alive (%) at day 24:** |
|---|---|---|---|
| Anti-DPI + CIS-DPI | 7/7 (100%) | 6/7 (86%) | 5/7 (71%) |
| Anti-DPI monotherapy | 6/8 (75%) | 5/8 (62%) | 3/7 (43%) |
| negative control | 4/7 (57%) | 1/7 (14%) | 0/7 (0%) |

### Example 7. Combination of cisplatin DPI with systemic chemotherapy and systemic immunotherapy

The protocol described in example 3 has been reproduced, except that three groups of 6-10 mice were compared:
(i) untreated negative control,
(ii) mice injected intraperitoneally (IP) with an anti-PD1 antibody (RMP 1-14, Bioceros, Utrecht, The Netherlands) in a two-week cycle (four administrations in weeks 1, week 2 is a week-off),
(iii) mice treated with a combination of cisplatin DPI at a dose of 0.5 mg/kg cisplatin and cisplatin IV at 1.5 mg/kg in a three-week cycle (three administration in weeks 1 and 3, week 2 is a week-off) and injected intra peritoneally (IP) with an anti-PD1 antibody (RMP 1-14, Bioceros, Utrecht, The Netherlands) in a two-week cycle (four administrations in weeks 1, week 2 is a week-off).

A decrease in tumor growth with the combination group (iii) compared with the untreated control group (i) and with group (ii) was observed as well as an increase in survival rates for the combination group (iii) compared with the untreated control group and group (ii) was observed.

### Example 8. Combination of cisplatin DPI with systemic chemotherapy and systemic immunotherapy

The protocol described in example 3 has been reproduced, except that three groups of 6-10 mice were compared:
(iv) untreated negative control,
(v) mice injected intraperitoneally (IP) with an anti-PD1 antibody (RMP 1-14, Bioceros, Utrecht, The Netherlands) in a two-week cycle (four administrations in weeks 1, week 2 is a week-off),
(vi) mice treated with a combination of cisplatin DPI at a dose of 0.5 mg/kg cisplatin and carboplatin IV at a cytotoxic dose in a three-week cycle (three administration in weeks 1 and 3, week 2 is a week-off) and injected intraperitoneally (IP) with an anti-PD1 antibody (RMP 1-14, Bioceros, Utrecht, The Netherlands) in a two-week cycle (four administrations in weeks 1, week 2 is a week-off).

A decrease in tumor growth with the combination group (iii) compared with the untreated control group and with group (ii) was observed as well as an increase in survival rates for the combination group (iii) compared with the untreated control group and group (ii) was observed.

### Example 9.- Combination of cisplatin DPI with systemic platinum doublets and systemic immunotherapy

The aim of this example is to demonstrate a synergistic effect between cisplatin DPI and immune checkpoint inhibitors (i.e. anti-PD1 immunotherapy), when combined with iv chemotherapy (i.e. systemic platinum doublet), which is the current standard of care in -60% stage IV NSCLC (PD-L1 biomarker expression < 50%)

Two main parameters were carefully followed during the study:
- The tumor growth over time by bioluminescence (in vivo optical imaging, the bioluminescence signal is directly proportional to the number of living M109 tumor cells in vivo). Bioluminescence analysis was performed two times a week, from day 3 (randomization) to day 38.
- Survival analysis, including Kaplan Meier survival curve and median survival (corresponding to the time where 50% mice died of their lung tumours or metastases.

The protocol described in example 3 has been reproduced, except that except that survival rates were also determined (no euthanasia on day 19 and no ex vivo bioluminescence analysis), the treatment started on day 6 according to the scheme table 7 below and that four groups of 8-24 mice were compared:
(i) Negative controls, i.e. saline iv injection + Anti-βGAL rat IgG2a ip injection (isotype control of anti-PD1, BioXCell, West Lebanon, NH, USA) + DPI vehicle (i.e. the powder without cisplatin, only the excipients),
(ii) mice injected ip with an anti-PD1 antibody (RMP 1-14, BioXCell, West Lebanon, NH, USA) and with a platinum doublet iv composed of 1.5 mg/kg cisplatin and 10 mg/kg paclitaxel in a one-week cycle for one cycle according to the scheme below,
(iii) mice treated with a combination of cisplatin DPI at a dose of 0.5 mg/kg cisplatin (from example 2) and the anti-PD1 antibody ip (RMP 1-14, BioXCell, West Lebanon, NH, USA) and 10 mg/kg paclitaxel iv (i.e. Taxol) in a one-week cycle for one cycle according to the scheme below.
(iv) mice treated with a combination of cisplatin DPI at a dose of 0.5 mg/kg cisplatin (from example 2) and the anti-PD1 antibody ip (RMP 1-14, BioXCell, West Lebanon, NH, USA) and platinum doublet iv composed of 1.5 mg/kg cisplatin and 10 mg/kg paclitaxel in a one-week cycle for one cycle .

**Table 7.- Dose regimen and scheme of administration followed in Example 9**

| **Group s:** | **i. Negative control** | **ii. anti-PD1 ip + platinum doublet iv** | **iii. anti-PD1 ip + Taxol iv + CIS DPI** | **iv. anti-PD1 ip + platinum doublet iv + CIS DPI** |
|---|---|---|---|---|
| **Day 0** | M109 cell engraftment | | | |
| **Day 3** | Randomization | | | |
| **Day 6** | Saline iv and Vehicle DPI | 1.5 mg/kg cisplatin iv and 10 mg/kg paclitaxel iv | 10 mg/kg paclitaxel iv and 0.5 mg/kg CIS DPI | 1.5 mg/kg cisplatin iv and 10 mg/kg paclitaxel iv and 0.5 mg/kg CIS DPI |
| **Day 7** | Isotype 5 mg/kg | Anti-PD1 5 mg/kg | Anti-PD1 5 mg/kg | Anti-PD1 5 mg/kg |
| **Day 8** | Isotype 5 mg/kg and Vehicle DPI | Anti-PD1 5 mg/kg | Anti-PD1 5 mg/kg and 0.5 mg/kg CIS DPI | Anti-PD1 5 mg/kg and 0.5 mg/kg CIS DPI |
| **Day 9** | Isotype 5 mg/kg | Anti-PD1 5 mg/kg | Anti-PD1 5 mg/kg | Anti-PD1 5 mg/kg |
| **Day 10** | Isotype 5 mg/kg and Vehicle DPI | Anti-PD1 5 mg/kg | Anti-PD1 5 mg/kg and 0.5 mg/kg CIS DPI | Anti-PD1 5 mg/kg and 0.5 mg/kg CIS DPI |

The mice that died during the endotracheal administration procedure were not considered in the analysis - 9/16 of negative controls, 5/14 of anti-PD1/Taxol/cisplatin DPI, and 13/24 of anti-PD1/Pt doublet/cisplatin DPI died during the administration procedure.

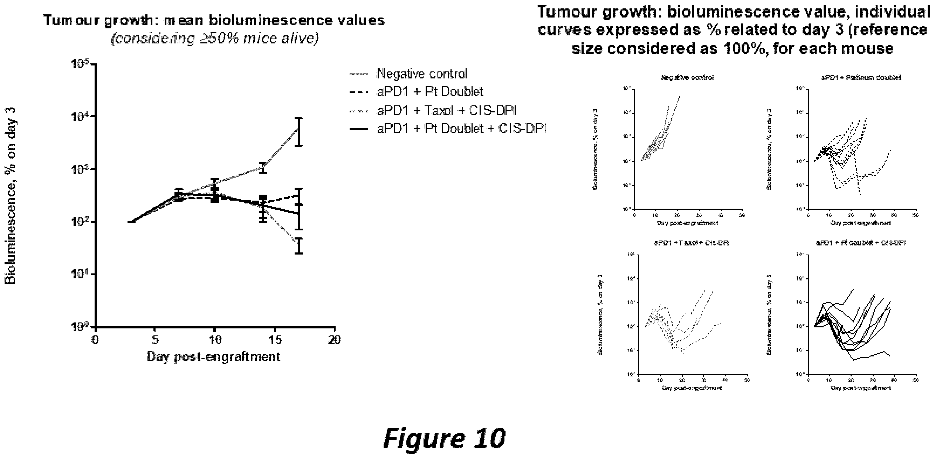

As presented in Figure 10, Cisplatin DPI contribution in the combination groups (i.e. groups iii and iv) allowed a better control of tumour growth, with a large majority of responders, which was more discernible than with the systemic chemotherapy/ immunotherapy (i.e. groups ii). From day 17 (i.e. 7 days after the last treatment administration), the curves separated, with a tumor re-growth observed in the standard of care group (group ii), while tumor cell number kept reducing in both the cisplatin DPI combination groups.
- Negative controls: not controlled and continuous tumor growth, with more than 500-fold increase of tumor cell number between day 3 and 21.
- Anti-PD1 + platinum doublet: 7/13 (54%) mice had temporary bioluminescence values below 100% (which indicates a reduction of tumor cell number compared to before the first treatment administration).
- Anti-PD1 + Taxol + cisplatin DPI: 7/9 (78%) mice had bioluminescence values below 100% indicating a reduction of tumour cell number compared to day 3.
- Anti-PD1 + platinum doublet + cisplatin DPI: 9/11 (82%) mice had bioluminescence values below 100% indicating a reduction of tumor cell number compared to day 3.

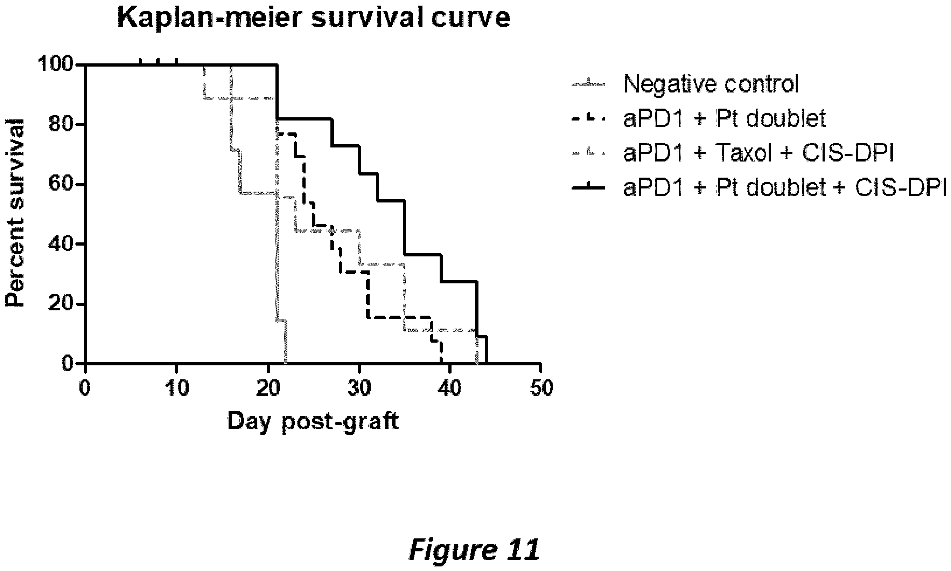

As it can be seen in figure 11 and in table 8.-, the addition of cisplatin DPI to the systemic anti-PD1/platinum doublet therapy significantly prolong the survival rates (p<0.05, log-rank test), with a median survival increase of 40% compared to the platinum doublet (35 vs 25 days, respectively).

**Table 8.- Survival rate at different timepoints**

| **Group** | **Mice alive (%) at day 25,** | **Mice alive (%) at day** **32_{:}** | **Mice alive (%) at day 42:** |
|---|---|---|---|
| (iv) antiPD1 + Pt doublet +CIS-DPI | 9/11 (82%) | 6/11 (56%) | 3/11 (27%) |
| (iii) antiPD1 + Taxol +CIS-DPI | 4/9 (44%) | 3/9 (33%) | 1/9 (11%) |
| (ii) antiPD1 + Pt doublet | 6/13 (46%) | 2/13 (15%) | 0/13 (0%) |
| (i) negative control | 0/7 (0%) | 0/7 (0%) | 0/7 (0%) |

### Example 10. Demonstration of the absence of environmental contamination with the kit for inhaled chemotherapy described in the invention (kit 1), compared with a conventional kit for inhalation.

The kit of the present invention was compared to a conventional kit in terms of environmental contamination.
a. Procedure to collect the environmental contamination from the use of a conventional kit composed with an RS.01 dry powder inhaler (RPC Plastiape, Osnago, Italy), a size 3 HPMC capsule (Quali-V-I, Qualicaps, Madrid, Spain) containing 20 mg cisplatin formulation (prepared in Example 2).

Briefly, a size 3 HPMC capsule (Quali-V-I, Qualicaps) was hand-filled with about 20 mg powder (prepared according to Example 2). The device was then prepared and use as directed in the instructions to the patient.

All the following preparation steps were performed over a glass crystallizer of about 20 cm diameter in order to collect the powder being at the origin of the environmental contamination. The capsule was placed into a RS.01 dry powder inhaler (RPC Plastiape), the device was closed and the capsule pierced. The device was placed in the apparatus described in the European Pharmacopeia 9.02 for dose collection. This apparatus is capable of quantitatively capturing the delivered dose. To simulate patient's inhalation, a flow rate of 100 ± 5 L/min measured using a DFM3 flow meter (Copley Scientific, Nottingham, UK) was obtained with two HCP5 air pumps (Copley Scientific, Nottingham, UK) connected in series to a TPK critical flow controller (Copley Scientific, Nottingham, UK). A 250 mL beaker was placed around the DPI device during activation of the pumps (i.e. during inhalation), to collect contaminant particles (i.e. air contamination). The critical flow controller was used to ensure a deposition time of 2.4 s at 100 L/min and a critical flow with a P3/P2 ratio < 0.5, as required by the European Pharmacopeia 9.02.

The device was opened, the capsule removed and the device reclosed. The powder fraction responsible for contamination during manipulation (i.e. the powder remaining in the crystallizer, the gloves of the investigator, from the device in open position and outside the capsule) was recovered with extensive washing with DMF and analyzed for its platinum content using the electrothermal atomic absorption spectrometry (ETAAS) validated method described by Levet et al (Levet et al, Int J Pharm 2016).

b. Procedure to collect the environmental contamination from the use of the kit 1 of the present invention composed with an adapted disposable RS.01 dry powder inhaler (Plastiape), a size 3 HPMC capsule (Quali-V-I, Qualicaps) containing 20 mg cisplatin formulation (prepared in Example 2). The adaptation of the RS.01 device consisted of being not re-openable once the capsule is pierced and was sealed; i.e. isolated from the environment, immediately after inhalation.

Briefly, a size 3 HPMC capsule (Quali-V-I, Qualicaps) was hand-filled with about 20 mg powder (prepared according to Example 1). The disposable DPI device was then prepared as directed in the instructions to the patient.

All the following preparation steps were performed over a glass crystallizer of about 20 cm diameter in order to collect the powder being at the origin of the environmental contamination. The capsule was placed into the disposable RS.01 dry powder inhaler (RPC Plastiape), the device was closed and the capsule pierced. The device was placed in the apparatus described in the European Pharmacopeia 9.02 for dose collection. This apparatus is capable of quantitatively capturing the delivered dose. To simulate patient's inhalation, a flow rate of 100 ± 5 L/min measured using a DFM3 flow meter (Copley Scientific, Nottingham, UK) was obtained with two HCP5 air pumps (Copley Scientific, Nottingham, UK) connected in series to a TPK critical flow controller (Copley Scientific, Nottingham, UK). A 250 mL beaker was placed around the DPI device during activation of the pumps (i.e. during inhalation), to collect contaminant particles (i.e. air contamination). The critical flow controller was used to ensure a deposition time of 2.4 s at 100 L/min and a critical flow with a P3/P2 ratio < 0.5, as required by the European Pharmacopeia 9.02.

The disposable DPI device was then immediately sealed, i.e. isolated from the environment.

The powder fraction responsible for contamination during manipulation (i.e. the powder remaining in the crystallizer, the gloves of the investigator and outside the device) was recovered with extensive washing with DMF and analyzed for its platinum content using the electrothermal atomic absorption spectrometry (ETAAS) validated method described by Levet et al (Levet et al, Int J Pharm 2016).

The cisplatin amount from air contamination was 4 ± 2 µg for the kit 1 (mean ± SD, n=6) which confirmed the ability of the adapted disposable DPI device according to the present invention to limit air contamination during inhalation.

As presented in Figure 13, the cisplatin powder fraction responsible for contamination during manipulation collected with the kit 1 of the present invention was significantly lower than with the conventional kit ((***) means p<0.001, Student t-test, n=3).

### Example 11. Demonstration of the absence of environmental contamination with the kit for inhaled chemotherapy described in the invention (kit 2), compared with a conventional kit for inhalation.

The kit of the present invention was compared to a conventional kit in terms of environmental contamination.
a. The same procedure as described in Example 10 was followed to collect the environmental contamination from the use of a conventional kit composed with an RS.01 dry powder inhaler (RPC Plastiape, Osnago, Italy), a size 3 HPMC capsule (Quali-V-I, Qualicaps, Madrid, Spain) containing 20 mg cisplatin formulation (prepared in Example 2).
b. Procedure to collect the environmental contamination from the use of the kit 2 of the present invention composed with an adapted disposable RS.01 dry powder inhaler (Plastiape), a size 3 HPMC capsule (Quali-V-I, Qualicaps) containing 20 mg cisplatin formulation (prepared in Example 2). The adaptation of the RS.01 device consisted of being not re-openable once the capsule is pierced, capping the drug exit during capsule piercing and inhalation by blocking any air exit through one-way valves and sealed, i.e. isolated from the environment, immediately after inhalation.

Briefly, a size 3 HPMC capsule (Quali-V-I, Qualicaps) was hand-filled with about 20 mg powder (prepared according to Example 1). The device was then prepared as directed in the instructions to the patient.

All the following preparation steps were performed over a glass crystallizer of about 20 cm diameter in order to collect the powder being at the origin of the environmental contamination. The capsule was placed into the disposable RS.01 dry powder inhaler (RPC Plastiape), the device was closed and the capsule pierced. The device was placed in the apparatus described in the European Pharmacopeia 9.02 for dose collection. This apparatus is capable of quantitatively capturing the delivered dose. To simulate patient's inhalation, a flow rate of 100 ± 5 L/min measured using a DFM3 flow meter (Copley Scientific, Nottingham, UK) was obtained with two HCP5 air pumps (Copley Scientific, Nottingham, UK) connected in series to a TPK critical flow controller (Copley Scientific, Nottingham, UK). A 250 mL beaker was placed around the DPI device during activation of the pumps (i.e. during inhalation), to collect contaminant particles (i.e. air contamination). The critical flow controller was used to ensure a deposition time of 2.4 s at 100 L/min and a critical flow with a P3/P2 ratio < 0.5, as required by the European Pharmacopeia 9.02.

The disposable device was then immediately sealed, i.e. isolated from the environment.

The powder fraction responsible for contamination during manipulation (i.e. the powder remaining in the crystallizer, the gloves of the investigator and outside the device) was recovered with extensive washing with DMF and analyzed for its platinum content using the electrothermal atomic absorption spectrometry (ETAAS) validated method described by Levet et al (Levet et al, Int J Pharm 2016).

Although the cisplatin amount from air contamination collected with the kit 1 for inhalation of the present invention was 4 ± 2 µg (mean ± SD, n=6), which confirmed the ability of DPI device to limit air contamination during inhalation, the cisplatin amount from air contamination with kit 2 of the invention was even much better than the kit 1 as it was below the limit of quantification of the ETAAS method.

More interestingly, the cisplatin powder fraction responsible for contamination during manipulation collected with the kit 1 and kit 2 of the present invention were both much lower than with the conventional kit.

It is to be understood that the present invention has been described using a mice model, while being destined to human or mammals. Accordingly, administration scheme, cycles and dose regimen described in the present examples are adapted to mice model and illustrates the human therapeutic plan.

For example, in mice, the cycle is a one-week cycle illustrating the three week cycle commonly used in chemotherapy in humans. In mice, the number of administration of the single hermetically packaged therapeutic dose per cycle is typically 3, while in human, it is merely foreseen that the number of administration of the single hermetically packaged therapeutic dose per cycle is typically up to 15 to 21. Likewise, in mice, the anti-neoplastic agent in each single hermetically packaged therapeutic dose is typically 0.5 mg/kg (of the animal) while in human, the anti-neoplastic agent in each single hermetically packaged therapeutic dose is typically between 1 and 5 mg/m² body surface (for cisplatin).

It should be understood that the present invention is not limited to the described embodiments and that variations can be applied without going outside of the scope of the appended claims.

## Claims

1. Kit for an inhaled chemotherapy comprising:
a) a series of single hermetically packaged therapeutic doses of a dry powder for inhalation containing at least one anti-neoplastic agent chosen in the group of platinum drugs such as carboplatin, cisplatin, oxaliplatin, or nedaplatin with a lipid matrix
b) a series of disposable dry powder inhaler devices,
wherein
i) each single hermetically packaged therapeutic dose having a closed state and an open state,
ii) each disposable dry powder inhaler device having a closed position and an open position,
iii) each single hermetically packaged therapeutic dose being provided for being accommodated in said closed state in one disposable dry powder inhaler device of said series of disposable dry powder inhaler devices,
iv) and each single disposable dry powder inhaler device is provided to be in said closed position before or once each single hermetically packaged therapeutic dose become in said open state,
wherein said single hermetically packaged therapeutic dose is a capsule or a blister and
wherein each disposable dry powder inhaler device comprises a dispersion chamber in fluid connection with an inhalation channel and connected to a loading chamber for accommodating said single hermetically packaged therapeutic dose inside said disposable dry powder inhaler device, said loading chamber comprising activation means having an activated position and a rest position, said activated position being provided to open said single hermetically packaged therapeutic dose and locking means provided to prevent extraction of the single hermetically packaged therapeutic dose once said activation means have been moved to said activated position, or said activated position being provided to open said single hermetically packaged therapeutic dose and locking means having an open position and a lock position provided to prevent extraction of the single hermetically packaged therapeutic dose, said activation means being provided to be able to be moved to said activated position only when said locking means are in said lock position, or said disposable dry powder inhaler device comprising closing means provided to isolate the outside environment from the inside of the disposable dry powder inhaler device, in particular by closing the inhalation channel.

2. Kit for a inhaled chemotherapy according to the claim 1, wherein the lipid matrix is a matrix of pure or a mixture of triglycerides, such as for example tristearin, hydrogenated vegetal oil (e.g. hydrogenated castor oil) or any hydrophobic glyceride derivative having values of Log P higher than 5, preferably comprised between 14 and 25, more particularly between 18 and 25 and preferably around 20, as measured using the shake-flask method.

3. The kit for an inhaled chemotherapy according to claim 1 or 2 wherein the lipid matrix is further comprising one or more excipient and/or a carrier.

4. The kit for an inhaled chemotherapy according to claim 3, wherein the excipient is a PEGylated excipient.

5. Kit for an inhaled chemotherapy according to the claim4 4, wherein the PEGylated excipient is derived from vitamin E or from phospholipids, such as tocopheryl polyethylene glycol succinate (TPGS) or distearoyl phosphoethanolamine polyethylene glycol 2000 (DSPE-mPEG-2000).

6. Kit for an inhaled chemotherapy according to any of the claims 1 to 5, wherein said disposable dry powder inhaler device is ready to use.

7. Kit for a inhaled chemotherapy according to any of the claims 1 to 6, wherein said single hermetically packaged therapeutic dose comprises a therapeutic dose in the form of a dry and fine powder having a geometric particle size distribution (PSD) d₅₀ lower than or equal to 30 µm, preferably lower than 15 µm, preferably lower than or equal to 10 µm, preferably lower than or equal to 5 µm, and a geometric particle size distribution (PSD) d₉₀ lower than or equal to 60 µm, preferably lower than or equal to 30 µm, more preferably lower than or equal to 15 µm, preferably lower than or equal to 10 µm and more preferably lower than or equal to 7 µm, and optionally a volume mean diameter D[4,3] lower than or equal to 40 µm, preferably lower than or equal to 20 µm, more preferably lower than or equal to 15 µm, preferably lower than or equal to 10 µm, preferably lower than or equal to 6 µm

8. Kit for an inhaled chemotherapy according to any of the claims 1 to 7, wherein each single hermetically packaged therapeutic dose comprises a total powder weight content between 2 to 100 mg, preferably between 3 and 80 mg, more preferably between 4 and 60 mg, in particular between 5 and 50 mg, such as for example between 6 and 20, or between 7 and 15, like around 10 mg, or comprises between 0.1 and 90 mg, preferably between 1 mg and 40 mg such as between 0.5 and 35 mg, preferably between 1 and 30 mg, in particular between 2 and 25 mg of said anti-neoplastic agent, or comprises from 0,1 to 99.9 mg, preferably between 1 and 49 mg of said lipid matrix, or comprises from 0.01 to 5 mg, preferably between 0.03 mg and 1 mg of PEGylated excipient.

9. Kit for an inhaled chemotherapy according to any of the claims 1 to 8, being suitable for use in a polytherapy, preferably said polytherapy comprises one therapy chosen in the group consisting of intravenous infusion or oral chemotherapy, immunotherapy, targeted therapy, hormonotherapy, tumor ablative surgery, ablative surgery for removing a part of or a full organ bearing a tumor, a curative surgery, a radiotherapy and their combination and at least one chemotherapy by inhalation as additional therapy.

10. Kit for an inhaled chemotherapy according to claim 9, wherein said further therapy comprises at least intravenous infusion or oral chemotherapy or an immunotherapy provided to be administered for a series of cycles, each cycle comprising an administration period and an off-period, said at least one chemotherapy by inhalation being provided to be administered in a period before said therapy and/or during said off-period.

11. Kit for an inhaled chemotherapy according to claim 10, wherein said administration period extends over less than one day, preferably over a period of time between 15 minutes to 6 hours, preferably between 30 minutes and 4 hours, such as between 1 and 3 hours, and where said off-period extends over a period of time from 2 to 5 weeks, preferably from 2 to 4 weeks, more preferably between 2,5 and 3,5 weeks and particularly over about 3 weeks + 2 days.

12. Kit for a inhaled chemotherapy according to any of the claims 9 to 11, wherein said at least one chemotherapy by inhalation is provided to be administered at a rate of at least 1 single hermetically packaged therapeutic dose of anti-neoplastic agent /week of period before said therapy and/or during said off-period, preferably of at least 3 single hermetically packaged therapeutic doses of anti-neoplastic agent /week of period before said therapy and/or during said off-period, more preferably of at least 5 single hermetically packaged therapeutic doses of anti-neoplastic agent /week of period before said therapy and/or during said off-period, possibly 7 single packaged therapeutic doses of anti-neoplastic agent /week of period before said therapy and/or during said off-period for a daily dosage regimen, optionally in an equally spread manner over said period before said therapy and/or during said off-period,

13. Kit for an inhaled chemotherapy according to claim 9 or claim 12, wherein said polytherapy comprises at least tumor ablative surgery, ablative surgery for removing a part of or a full organ bearing a tumor, a curative surgery, or a radiotherapy followed by a recovery period, said at least one chemotherapy by inhalation being provided to be administered on a period before, during or after said therapy.

14. Kit for a inhaled chemotherapy according to claim 13, wherein said at least one chemotherapy by inhalation is provided to be administered at a rate of at least 1 single hermetically packaged therapeutic dose of anti-neoplastic agent /week of period before, during or after said therapy, preferably of at least 3 single hermetically packaged therapeutic doses of anti-neoplastic agent/week of period before, during or after said therapy, more preferably of at least 5 single hermetically packaged therapeutic doses of anti-neoplastic agent/week of period before, during or after said therapy, possibly 7 single packaged therapeutic doses of anti-neoplastic agent/week of period before, during or after said therapy for a daily dosage regimen, optionally in an equally spread manner over said period before, during or after said therapy.

## Patentansprüche

1. Kit für eine inhalative Chemotherapie, umfassend:
a) eine Reihe von einzelnen hermetisch verpackten therapeutischen Dosen eines Trockenpulvers zur Inhalation, das mindestens ein anti-neoplastisches Mittel aus der Gruppe der Platinmedikamente wie Carboplatin, Cisplatin, Oxaliplatin oder Nedaplatin mit einer Lipidmatrix enthält
b) eine Reihe von Einwegtrockenpulverinhalatorvorrichtungen,
wobei
i) jede einzelne hermetisch verpackte therapeutische Dosis einen geschlossenen und einen offenen Zustand aufweist,
ii) jede Einwegtrockenpulverinhalatorvorrichtung eine geschlossene Position und eine offene Position aufweist,
iii) jede einzelne hermetisch verpackte therapeutische Dosis bereitgestellt wird, um in dem geschlossenen Zustand in einer Einwegtrockenpulverinhalatorvorrichtung der Reihe von Einwegtrockenpulverinhalatorvorrichtungen aufgenommen zu werden,
iv) und jede einzelne Einwegtrockenpulverinhalatorvorrichtung bereitgestellt wird, um in der geschlossenen Position zu sein, bevor oder wenn jede einzelne hermetisch verpackte therapeutische Dosis in den offenen Zustand kommt,
wobei die einzelne hermetisch verpackte therapeutische Dosis eine Kapsel oder ein Blister ist und
wobei jede Einwegtrockenpulverinhalatorvorrichtung eine Dispersionskammer umfasst, die in Fluidverbindung mit einem Inhalationskanal steht und zum Aufnehmen der einzelnen hermetisch verpackten therapeutischen Dosis innerhalb der Einwegtrockenpulverinhalatorvorrichtung mit einer Ladekammer verbunden ist, die Ladekammer umfassend Aktivierungsmittel, die eine Aktivierungsposition und eine Ruheposition aufweisen, wobei die Aktivierungsposition bereitgestellt wird, um die einzelne hermetisch verpackte therapeutische Dosis zu öffnen, und Verriegelungsmittel, die bereitgestellt werden, um das Extrahieren der einzelnen hermetisch verpackten therapeutischen Dosis zu verhindern, wenn die Aktivierungsmittel in die Aktivierungsposition bewegt worden sind, oder wobei die Aktivierungsposition bereitgestellt wird, um die einzelne hermetisch verpackte therapeutische Dosis zu öffnen, und Verriegelungsmittel eine Öffnungsposition und eine Verriegelungsposition aufweisen, die bereitgestellt werden, um das Extrahieren der einzelnen hermetisch verpackten therapeutischen Dosis zu verhindern, wobei die Aktivierungsmittel bereitgestellt werden, um nur dann in die Aktivierungsposition bewegt werden zu können, wenn sich die Verriegelungsmittel in der Verriegelungsposition befinden, oder die Einwegtrockenpulverinhalatorvorrichtung umfassend Verriegelungsmittel, die bereitgestellt werden, um die Außenumgebung vom Inneren der Einwegtrockenpulverinhalatorvorrichtung zu isolieren, insbesondere durch Schließen des Inhalationskanals.

2. Kit für eine inhalative Chemotherapie nach Anspruch 1, wobei die Lipidmatrix eine Matrix aus reinen oder einer Mischung von Triglyceriden, wie zum Beispiel Tristearin, hydriertem Pflanzenöl (z. B. hydriertem Rizinusöl) oder irgendeinem hydrophoben Glyceridderivat ist, die Log P-Werte größer als 5 aufweist, die vorzugsweise zwischen 14 und 25, insbesondere zwischen 18 und 25 und vorzugsweise um 20 umfasst sind, wie unter Verwendung des Schüttelkolbenverfahrens gemessen.

3. Kit für eine inhalative Chemotherapie nach Anspruch 1 oder 2, wobei die Lipidmatrix ferner einen oder mehrere Hilfsstoffe und/oder einen Träger umfasst.

4. Kit für eine inhalative Chemotherapie nach Anspruch 3, wobei der Hilfsstoff ein PEGylierter Hilfsstoff ist.

5. Kit für eine inhalative Chemotherapie nach Anspruch 4, wobei der PEGylierte Hilfsstoff von Vitamin E oder von Phospholipiden, wie Tocopherylpolyethylenglykolsuccinat (TPGS) oder Distearoylphosphoethanolaminpolyethylenglykol 2000 (DSPE-mPEG-2000), abgeleitet ist.

6. Kit für eine inhalative Chemotherapie nach einem der Ansprüche 1 bis 5, wobei die Einwegtrockenpulverinhalatorvorrichtung zur Verwendung bereit ist.

7. Kit für eine inhalative Chemotherapie nach einem der Ansprüche 1 bis 6, wobei die einzelne hermetisch verpackte therapeutische Dosis eine therapeutische Dosis in Form eines trockenen und feinen Pulvers umfasst, das eine geometrische Partikelgrößenverteilung (PSD) d₅₀ von weniger als oder gleich 30 µm, vorzugsweise weniger als oder gleich 15 µm, vorzugsweise weniger als oder gleich 10 µm, vorzugsweise weniger als oder gleich 5 µm, und eine geometrische Partikelgrößenverteilung (PSD) d₉₀ von weniger als oder gleich 60 µm, vorzugsweise weniger als oder gleich 30 µm, noch bevorzugter weniger als oder gleich 15 µm, vorzugsweise weniger als oder gleich 10 µm und noch bevorzugter weniger als oder gleich 7 µm, und optional einen mittleren Volumenmitteldurchmesser D[4,3] von weniger als oder gleich 40 µm, vorzugsweise weniger als oder gleich 20 µm, noch bevorzugter weniger als oder gleich 15 µm, vorzugsweise weniger als oder gleich 10 µm, vorzugsweise weniger als oder gleich 6 µm aufweist

8. Kit für eine inhalative Chemotherapie nach einem der Ansprüche 1 bis 7, wobei jede einzelne hermetisch verpackte therapeutische Dosis einen Gesamtpulvergewichtsgehalt zwischen 2 und 100 mg, vorzugsweise zwischen 3 und 80 mg, noch bevorzugter zwischen 4 und 60 mg, insbesondere zwischen 5 und 50 mg, wie zum Beispiel zwischen 6 und 20, oder zwischen 7 und 15, wie um 10 mg, umfasst, oder zwischen 0,1 und 90 mg, vorzugsweise zwischen 1 mg und 40 mg, wie beispielsweise zwischen 0,5 und 35 mg, vorzugsweise zwischen 1 und 30 mg, insbesondere zwischen 2 und 25 mg des antineoplastischen Mittels umfasst, oder von 0,1 bis 99,9 mg, vorzugsweise zwischen 1 und 49 mg der Lipidmatrix umfasst, oder 0,01 bis 5 mg, insbesondere zwischen 0,03 mg und 1 mg des PEGylierten Hilfsstoffs umfasst.

9. Kit für eine inhalative Chemotherapie nach einem der Ansprüche 1 bis 8, das zur Verwendung in einer Polytherapie geeignet ist, wobei die Polytherapie vorzugsweise eine Therapie umfasst, die aus der Gruppe ausgewählt ist, die aus intravenöser Infusion oder oraler Chemotherapie, Immuntherapie, zielgerichteter Therapie, Hormontherapie, tumorablativer Chirurgie, ablativer Chirurgie zum Entfernen eines Teils oder eines ganzen Organs, das einen Tumor trägt, einer kurativen Chirurgie, einer Strahlentherapie und deren Kombination und mindestens einer Chemotherapie durch Inhalation als zusätzliche Therapie besteht.

10. Kit für eine inhalative Chemotherapie nach Anspruch 9, wobei die weitere Therapie mindestens eine intravenöse Infusion oder eine orale Chemotherapie oder eine Immuntherapie umfasst, die bereitgestellt wird, um für eine Reihe von Zyklen verabreicht zu werden, jeder Zyklus umfassend einen Verabreichungszeitraum und einen Aus-Zeitraum, wobei die mindestens eine Chemotherapie durch Inhalation bereitgestellt wird, um in einem Zeitraum vor der Therapie und/oder während des Aus-Zeitraums verabreicht zu werden.

11. Kit für eine inhalative Chemotherapie nach Anspruch 10, wobei sich der Verabreichungszeitraum über weniger als einen Tag, vorzugsweise über einen Zeitraum zwischen 15 Minuten und 6 Stunden, vorzugsweise zwischen 30 Minuten und 4 Stunden, wie beispielsweise zwischen 1 und 3 Stunden, erstreckt und wobei sich der Aus-Zeitraum über einen Zeitraum von 2 bis 5 Wochen, vorzugsweise von 2 bis 4 Wochen, noch bevorzugter zwischen 2,5 und 3,5 Wochen und insbesondere über etwa 3 Wochen ± 2 Tage erstreckt.

12. Kit für eine inhalative Chemotherapie nach einem der Ansprüche 9 bis 11, wobei die mindestens eine Chemotherapie durch Inhalation bereitgestellt wird, um mit einer Rate von mindestens 1 einzelnen hermetisch verpackten therapeutischen Dosis eines antineoplastischen Mittels/Woche des Zeitraums vor der Therapie und/oder während des Aus-Zeitraums, vorzugsweise von mindestens 3 einzelnen hermetisch verpackten therapeutischen Dosen eines antineoplastischen Mittels/Woche des Zeitraums vor der Therapie und/oder während des Aus-Zeitraums, vorzugsweise von mindestens 5 einzelnen hermetisch verpackten therapeutischen Dosen eines antineoplastischen Mittels/Woche des Zeitraums vor der Therapie und/oder während des Aus-Zeitraums, möglicherweise von 7 einzelnen verpackten therapeutischen Dosen eines antineoplastischen Mittels/Woche des Zeitraums vor der Therapie und/oder während des Aus-Zeitraums für ein tägliches Dosierungsregime, optional in gleichmäßiger Verteilung über den Zeitraum vor der Therapie und/oder während des Aus-Zeitraums, verabreicht zu werden,

13. Kit für eine inhalative Chemotherapie nach Anspruch 9 oder Anspruch 12, wobei die Polytherapie mindestens eine tumorablative Chirurgie, eine ablative Chirurgie zum Entfernen eines Teils oder eines ganzen Organs, das einen Tumor trägt, eine kurative Chirurgie oder eine Strahlentherapie, gefolgt von einem Erholungszeitraum, umfasst, wobei die mindestens eine Chemotherapie durch Inhalation bereitgestellt wird, um in einem Zeitraum vor, während oder nach der Therapie verabreicht zu werden.

14. Kit für eine inhalative Chemotherapie nach Anspruch 13, wobei die mindestens eine Chemotherapie durch Inhalation bereitgestellt wird, um mit einer Rate von mindestens 1 einzelnen hermetisch verpackten therapeutischen Dosis eines antineoplastischen Mittels/Woche des Zeitraums vor, während oder nach der Therapie, vorzugsweise von mindestens 3 einzelnen hermetisch verpackten therapeutischen Dosen eines antineoplastischen Mittels/Woche des Zeitraums vor, während oder nach der Therapie, noch bevorzugter von mindestens 5 einzelnen hermetisch verpackten therapeutischen Dosen eines antineoplastischen Mittels/Woche des Zeitraums vor, während oder nach der Therapie, möglicherweise von 7 einzelnen hermetisch verpackten therapeutischen Dosen eines antineoplastischen Mittels/Woche des Zeitraums vor, während oder nach der Therapie für ein tägliches Dosierungsregime, optional in gleichmäßiger Verteilung über den Zeitraum vor, während oder nach der Therapie, verabreicht zu werden.

## Revendications

1. Kit de chimiothérapie inhalée comprenant :
a) une série de doses thérapeutiques uniques emballées hermétiquement d'une poudre sèche pour inhalation contenant au moins un agent antinéoplasique choisi dans le groupe des médicaments à base de platine tels que le carboplatine, le cisplatine, l'oxaliplatine ou le nédaplatine avec une matrice lipidique
b) une série de dispositifs d'inhalation de poudre sèche jetables,
dans lequel
i) chaque dose thérapeutique unique emballée hermétiquement ayant un état fermé et un état ouvert,
ii) chaque dispositif d'inhalation de poudre sèche jetable ayant une position fermée et une position ouverte,
iii) chaque dose thérapeutique unique emballée hermétiquement étant prévue pour être logée dans ledit état fermé dans un dispositif d'inhalation de poudre sèche jetable de ladite série de dispositifs d'inhalation de poudre sèche jetables,
iv) et chaque dispositif d'inhalation de poudre sèche jetable unique est prévu pour être dans ladite position fermée avant ou une fois que chaque dose thérapeutique unique emballée hermétiquement passe audit état ouvert,
dans lequel ladite dose thérapeutique unique emballée hermétiquement est une capsule ou un blister et
dans lequel chaque dispositif d'inhalation de poudre sèche jetable comprend une chambre de dispersion en connexion fluide avec un canal d'inhalation et connectée à une chambre de chargement pour loger ladite dose thérapeutique unique emballée hermétiquement à l'intérieur dudit dispositif d'inhalation de poudre sèche jetable, ladite chambre de chargement comprenant des moyens d'activation ayant une position activée et une position de repos, ladite position activée étant prévue pour ouvrir ladite dose thérapeutique unique emballée hermétiquement et des moyens de verrouillage étant prévus pour empêcher l'extraction de la dose thérapeutique unique emballée hermétiquement une fois que lesdits moyens d'activation ont été déplacés vers ladite position activée, ou ladite position activée étant prévue pour ouvrir ladite dose thérapeutique unique emballée hermétiquement et lesdits moyens de verrouillage ayant une position ouverte et une position de verrouillage prévue pour empêcher l'extraction de la dose thérapeutique unique emballée hermétiquement, lesdits moyens d'activation étant prévus pour pouvoir être déplacés vers ladite position activée uniquement lorsque lesdits moyens de verrouillage sont dans ladite position de verrouillage, ou ledit dispositif d'inhalation de poudre sèche jetable comprenant des moyens de fermeture prévus pour isoler l'environnement extérieur de l'intérieur du dispositif d'inhalation de poudre sèche jetable, en particulier en fermant le canal d'inhalation.

2. Kit de chimiothérapie inhalée selon la revendication 1, dans lequel la matrice lipidique est une matrice de triglycérides purs ou un mélange de triglycérides, tels que par exemple la tristéarine, l'huile végétale hydrogénée (p. ex. l'huile de ricin hydrogénée) ou tout dérivé de glycéride hydrophobe ayant des valeurs de Log P supérieures à 5, de préférence comprises entre 14 et 25, plus particulièrement entre 18 et 25 et de préférence autour de 20, telles que mesurées à l'aide du procédé du flacon agité.

3. Kit de chimiothérapie inhalée selon la revendication 1 ou 2, dans lequel la matrice lipidique comprend en outre un ou plusieurs excipients et/ou un vecteur.

4. Kit de chimiothérapie inhalée selon la revendication 3, dans lequel l'excipient est un excipient PEGylé.

5. Kit de chimiothérapie inhalée selon la revendication 4, dans lequel l'excipient PEGylé est dérivé de la vitamine E ou de phospholipides, tels que le succinate polyéthylène glycol de tocophérol (TPGS) ou le distéaroyl phosphoéthanolamine polyéthylène glycol 2000 (DSPE-mPEG-2000).

6. Kit de chimiothérapie inhalée selon l'une quelconque des revendications 1 à 5, dans lequel ledit dispositif d'inhalation de poudre sèche jetable est prêt à l'emploi.

7. Kit de chimiothérapie inhalée selon l'une quelconque des revendications 1 à 6, dans lequel ladite dose thérapeutique unique emballée hermétiquement comprend une dose thérapeutique sous forme de poudre sèche et fine ayant une distribution granulométrique (PSD) géométrique d₅₀ inférieure ou égale à 30 µm, de préférence inférieure à 15 µm, de préférence inférieure ou égale à 10 µm, de préférence inférieure ou égale à 5 µm, et une distribution granulométrique (PSD) géométrique d₉₀ inférieure ou égale à 60 µm, de préférence inférieure ou égale à 30 µm, plus préférentiellement inférieure ou égale à 15 µm, de préférence inférieure ou égale à 10 µm et plus préférentiellement inférieure ou égale à 7 µm, et facultativement un diamètre moyen en volume D[4,3] inférieur ou égal à 40 µm, de préférence inférieur ou égal à 20 µm, plus préférentiellement inférieur ou égal à 15 µm, de préférence inférieur ou égal à 10 µm, de préférence inférieur ou égal à 6 µm

8. Kit de chimiothérapie inhalée selon l'une quelconque des revendications 1 à 7, dans lequel chaque dose thérapeutique unique emballée hermétiquement comprend une teneur en poids total en poudre comprise entre 2 et 100 mg, de préférence entre 3 et 80 mg, plus préférentiellement entre 4 et 60 mg, en particulier entre 5 et 50 mg, comme par exemple entre 6 et 20, ou entre 7 et 15, comme environ 10 mg, ou comprend entre 0,1 et 90 mg, de préférence entre 1 mg et 40 mg comme entre 0,5 et 35 mg, de préférence entre 1 et 30 mg, en particulier entre 2 et 25 mg dudit agent antinéoplasique, ou comprend de 0,1 à 99,9 mg, de préférence entre 1 et 49 mg de ladite matrice lipidique, ou comprend de 0,01 à 5 mg, de préférence entre 0,03 mg et 1 mg d'excipient PEGylé.

9. Kit de chimiothérapie inhalée selon l'une quelconque des revendications 1 à 8, convenant à une utilisation en polythérapie, ladite polythérapie comprenant de préférence une thérapie choisie dans le groupe consistant en une perfusion intraveineuse ou une chimiothérapie orale, une immunothérapie, une thérapie ciblée, une hormonothérapie, une chirurgie par ablation tumorale, une chirurgie par ablation pour l'ablation d'une partie ou de l'ensemble d'un organe porteur d'une tumeur, une chirurgie curative, une radiothérapie et leur combinaison et au moins une chimiothérapie par inhalation en tant que thérapie supplémentaire.

10. Kit de chimiothérapie inhalée selon la revendication 9, dans lequel ladite thérapie ultérieure comprend au moins une perfusion intraveineuse ou une chimiothérapie orale ou une immunothérapie prévue pour être administrée pour une série de cycles, chaque cycle comprenant une période d'administration et une période de repos, ladite au moins une chimiothérapie par inhalation étant prévue pour être administrée dans une période avant ladite thérapie et/ou pendant ladite période de repos.

11. Kit de chimiothérapie inhalée selon la revendication 10, dans lequel ladite période d'administration s'étend sur moins d'un jour, de préférence sur une période de temps comprise entre 15 minutes et 6 heures, de préférence entre 30 minutes et 4 heures, comme entre 1 et 3 heures, et où ladite période de repos s'étend sur une période de temps de 2 à 5 semaines, de préférence de 2 à 4 semaines, plus préférentiellement entre 2,5 et 3,5 semaines et notamment sur environ 3 semaines + 2 jours.

12. Kit de chimiothérapie inhalée selon l'une quelconque des revendications 9 à 11, dans lequel ladite au moins une chimiothérapie par inhalation est prévue pour être administrée à un taux d'au moins 1 dose thérapeutique unique emballée hermétiquement d'agent antinéoplasique / semaine de période avant ladite thérapie et/ou pendant ladite période de repos, de préférence d'au moins 3 doses thérapeutiques uniques emballées hermétiquement d'agent antinéoplasique / semaine de période avant ladite thérapie et/ou pendant ladite période de repos, plus préférentiellement d'au moins 5 doses thérapeutiques uniques emballées hermétiquement d'agent antinéoplasique / semaine de période avant ladite thérapie et/ou pendant ladite période de repos, éventuellement 7 doses thérapeutiques uniques emballées d'agent antinéoplasique / semaine de période avant ladite thérapie et/ou pendant ladite période de repos pour un schéma posologique journalier, facultativement de manière également répartie sur ladite période avant ladite thérapie et/ou pendant ladite période de repos.

13. Kit de chimiothérapie inhalée selon la revendication 9 ou la revendication 12, dans lequel ladite polythérapie comprend au moins une chirurgie par ablation tumorale, une chirurgie par ablation pour l'ablation d'une partie ou de la totalité d'un organe complet portant une tumeur, une chirurgie curative, ou une radiothérapie suivie d'une période de récupération, ladite au moins une chimiothérapie par inhalation étant prévue pour être administrée sur une période avant, pendant ou après ladite thérapie.

14. Kit de chimiothérapie inhalée selon la revendication 13, dans lequel ladite au moins une chimiothérapie par inhalation est prévue pour être administrée à un taux d'au moins 1 dose thérapeutique unique emballée hermétiquement d'agent antinéoplasique / semaine de période avant, pendant ou après ladite thérapie, de préférence d'au moins 3 doses thérapeutiques uniques emballées hermétiquement d'agent antinéoplasique / semaine de période avant, pendant ou après ladite thérapie, plus préférentiellement d'au moins 5 doses thérapeutiques uniques emballées hermétiquement d'agent antinéoplasique / semaine de période avant, pendant ou après ladite thérapie, éventuellement 7 doses thérapeutiques uniques emballées d'agent antinéoplasique / semaine de période avant, pendant ou après ladite thérapie pour un schéma posologique quotidien, facultativement de manière également répartie sur ladite période avant, pendant ou après ladite thérapie.
